(19) Europäisches Patentamt
European Patent Office
Office européen des brevets

(11) EP 0 623 608 B1

(12) **EUROPEAN PATENT SPECIFICATION**

(45) Date of publication and mention of the grant of the patent:
**30.05.2001 Bulletin 2001/22**

(21) Application number: **93902518.5**

(22) Date of filing: **22.01.1993**

(51) Int Cl.$^7$: **C07D 311/22**, A61K 31/35, C07D 311/58, C07D 311/96

(86) International application number:
**PCT/JP93/00078**

(87) International publication number:
**WO 93/15065 (05.08.1993 Gazette 1993/19)**

(54) **BENZOPYRAN DERIVATIVE, PRODUCTION THEREOF, AND PHARMACEUTICAL PREPARATION CONTAINING THE SAME AS ACTIVE INGREDIENT**

BENZOPYRAN-DERIVATE, DEREN HERSTELLUNG SOWIE DIESE ALS AKTIVE BESTANDTEILE ENTHALTENDE ARZNEIMITTEL

DERIVE DE BENZOPYRANE, PRODUCTION DE CE DERIVE ET PREPARATION PHARMACEUTIQUE LE CONTENANT COMME INGREDIENT ACTIF

(84) Designated Contracting States:
**AT BE CH DE DK ES FR GB IT LI NL SE**

(30) Priority: **24.01.1992 JP 1108692**
**24.01.1992 JP 1108792**
**11.09.1992 JP 24336692**

(43) Date of publication of application:
**09.11.1994 Bulletin 1994/45**

(73) Proprietor: **TEIJIN LIMITED**
**Osaka-shi, Osaka 541 (JP)**

(72) Inventors:
- **SHIOTA, Tatsuki**
  **Sagamihara-shi, Kanagawa (JP)**
- **TAKEYASU, Takumi**
  **Hino-shi, Tokyo 191 (JP)**
- **MOCHIZUKI, Tsutomu**
  **Hino-shi, Tokyo 191 (JP)**
- **KATAOKA, Kenichiro**
  **Tama-ku, Kawasaki-shi, Kanagawa (JP)**
- **TANABE, Hirofumi**
  **Hino-shi, Tokyo 191 (JP)**
- **OTA, Mikio**
  **Hino-shi, Tokyo 191 (JP)**
- **KANOH, Masatoshi**
  **Hino-shi, Tokyo 191 (JP)**
- **YAMAGUCHI, Hisao**
  **Hino-shi, Tokyo 191 (JP)**

(74) Representative: **Harrison, Ivor Stanley**
**Withers & Rogers,**
**Goldings House, 2 Hays Lane**
**London SE1 2HW (GB)**

(56) References cited:
**EP-A- 0 173 516**
**EP-A- 0 546 177**
**WO-A-92/01681**
**JP-A- 61 050 977**
**JP-B- 60 015 626**

- **JOURNAL OF MEDICINAL CHEMISTRY, vol.13, no.3, 1970, WASHINGTON US pages 584 - 585 G.LETTIERI ET AL. 'SUBSTITUTED CHROMAN-6-YL UREAS'**

Remarks:
The file contains technical information submitted after the application was filed and not included in this specification

EP 0 623 608 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

TECHNICAL FIELD

The present invention relates to novel benzopyran derivatives, processes for producing the same, the applications thereof as drugs, and pharmaceutical compositions containing the same. More specifically, it relates to a benzopyran derivative having on its aromatic ring an acylamino group, and pharmacologically acceptable acid adducts thereof, processes for production for the same, pharmaceutical compositions containing the same, and applications of these as drugs against hyperlipidemia.

BACKGROUND ART

Atherosclerosis is known to be an extremely important factor in various cardiovascular diseases. Research is actively underway with the aim of suppression of the progress of artherosclerosis or regression of atherosclerosis. In particular, while drugs for lowering cholesterol in the serum or arterial wall are recognised as being effective, no ideal drug has been ralized which has a significant clinical effect and few side effects.
On the other hand, it has become apparent that the accumulation of cholesterol esters in the arterial wall is an important factor in the progress of atherosclerosis.
Further, it is known that Acyl-Coenzyme A: Cholesterol Acyltransferase (ACAT) plays an important role in the formation of cholesterol esters in the tunica mucosa intestini tenuis or arterial wall.
In the past, urea derivatives (reference: J. Med. Chem., vol. 29, p. 1131 (1986), Japanese Unexamined Patent Publication (Kokai) No. 63-316761, Japanese Unexamined Patent Publication (Kokai) No. 1-93569, etc.) and amide derivatives (reference: Japanese Unexamined Patent Publication (Kokai) No. 63-253060 etc.), for example, have been reported as ACAT enzyme inhibitors.
On the other hand, as reports relating to benzopyran derivatives, there are the specification of U.S. Patent No. 4415741, the published specification of European Patent Application No. 485984, etc., but there is no specific disclosure on the compounds of the present invention among these and it is not known from them that the benzopyran derivatives have an ACAT inhibitory activity. Further, 6-acylamino-chromanones having anti-hyperlipidemia activity (reference: Japanese Examined Patent Publication (Kokoku) No. 60-15626 and Japanese Examined Patent Publication (Kokoku) No. 60-35346) are known, but they have a weaker action than the compounds of the present invention and it is not known that they have an ACAT inhibitory activity. Further, there are known chromans having urea groups on the aromatic ring (reference: J. Med. Chem., vol. 13, p. 584 (1970)), 8-acylaminobenzopyrans having a leukotriene antagonist activity (reference: Japanese Unexamined Patent Publication (Kokai) No. 61-50977, Japanese Unexamined Patent Publication (Kokai) No. 61-126061, Japanese Unexamined Patent Publication (Kokai) No. 61-143371, Japanese Unexamined Patent Publication (Kokai) No. 62-230760, J. Med. Chem., vol. 31, p. 84 (1988)), and 8-acylaminochromans having a local anesthetic action (reference: Khim. Getrotsikl. Soedin., 320 (1987)), but these are not known to have an anti-hyperlipidemia activity and ACAT inhibitory activity.

DISCLOSURE OF THE INVENTION

The object of the present invention is to provide novel benzopyran derivatives and processes for producing the same and, further, to provide pharmaceutical compositions having these derivatives as an effective ingredient and drugs against hyperlipidemia.
In accordance with the present invention, there are provided benzopyran derivatives and their pharmacologically acceptable acid adducts of the formula (I):

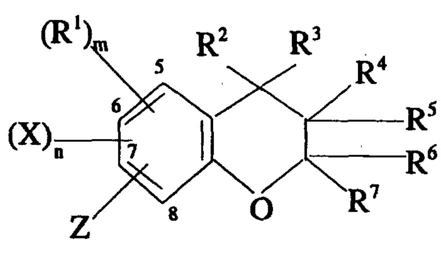

... (I)

wherein

X independently represents an unsubstituted or substituted $C_7$ to $C_{20}$ alkyl group, unsubstituted or substituted $C_7$ to $C_{20}$ alkoxy group, unsubstituted or substituted $C_7$ to $C_{20}$ acylamino group, unsubstituted or substituted $C_{13}$ to $C_{30}$ alkylamino group, unsubstituted or substituted $C_8$ to $C_{20}$ alkyloxycarbonyl group, unsubstituted or substituted $C_8$ to $C_{20}$ acyl group, unsubstituted or substituted $C_{13}$ to $C_{20}$ acyloxy group, unsubstituted or substituted $C_6$ to $C_{20}$ aryl group, or unsubstituted or substituted $C_6$ to $C_{20}$ aryloxy group, in which when X is substituted the substituent group is selected from halogen, hydroxy, amino, alkoxy and aryloxy groups;
Z represents the group:

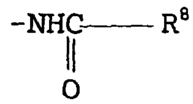

wherein $R^1$ independently represents a hydrogen atom, halogen atom, unsubstituted or substituted $C_1$ to $C_6$ lower alkyl group, unsubstituted or substituted $C_1$ to $C_6$ lower alkoxy group, hydroxyl group, nitro group, cyano group, unsubstituted or substituted $C_1$ to $C_6$ lower acylamino group, unsubstituted or substituted $C_1$ to $C_{12}$ alkylamino group, unsubstituted or substituted $C_1$ to $C_7$ lower alkyloxycarbonyl group, carboxyl group, unsubstituted or substituted $C_1$ to $C_7$ lower acyl group, or unsubstituted or substituted $C_1$ to $C_{12}$ acyloxy group, in which when $R^1$ is substituted the substituent group is selected from halogen, hydroxy and amino groups;
$R^2$ and $R^3$ together represent an oxygen atom;
$R^4$ to $R^7$ each represent a hydrogen atom, or
$R^4$ and $R^6$ are combined together to form a carbon-carbon bond;
$R^8$ represents a $C_5$ to $C_{20}$ cycloalkyl or cycloalkenyl group, an unsubstituted or substituted $C_6$ to $C_{20}$ aryl group, in which the substituent group is selected from halogen, $C_1$ to $C_{14}$ alkyl, $C_1$ to $C_{14}$ alkoxy, acylamino, mono- or di-alkylamino, alkoxycarbonyl, acyl, and acyloxy groups, which may be substituted with substituents selected from halogen, hydroxy, nitro, cyano, $C_1$ to $C_{10}$ alkoxy, $C_6$ to $C_{10}$ aryloxy and $C_1$ to $C_{12}$ alkylamino groups, or $R_8$ represents a group of the formula (VII):

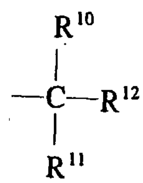

(VII)

wherein, $R^{10}$ and R" independently represent a hydrogen atom, or $C_1$ to $C_6$ lower alkyl group, $R^{12}$ represents a hydrogen atom, unsubstituted or substituted $C_1$ to $C_{19}$ alkyl or alkenyl group, unsubstituted or substituted $C_6$ to $C_{19}$ aryl group, unsubstituted or substituted $C_6$ to $C_{19}$ aryl alkyl group, or group of the formula -A-Z-B, wherein A represents a $C_1$ to $C_{12}$ alkyl group, Z represents an oxygen atom or sulfur atom, and B represents an unsubstituted $C_1$ to $C_{19}$ alkyl group, unsubstituted or substituted $C_6$ to $C_{19}$ aryl group, or unsubstituted or substituted $C_6$ to $C_{19}$ aryl alkyl group in which when $R^{12}$ or B is substituted the substituent group is selected from halogen, $C_1$ to $C_6$ alkyl, $C_1$ to $C_6$ alkoxy, hydroxy, nitro, cyano, $C_1$ to $C_6$ acylamino, $C_1$ to $C_{12}$ alkylamino, $C_1$ to $C_7$ alkoxycarbonyl, carboxyl, $C_1$ to $C_7$ acyl and $C_1$ to $C_{12}$ acyloxycarbonyl groups or $R^8$ represents a group of formula (IX):

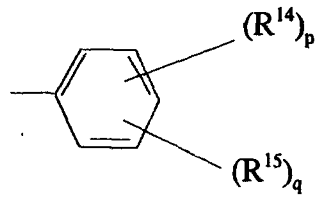

(IX)

wherein $R^{14}$ independently represents a substituted $C_1$ to $C_{14}$ alkyl group, substituted $C_1$ to $C_{14}$ alkoxy group, substituted $C_1$ to $C_{14}$ acylamino group, substituted $C_1$ to $C_{14}$ alkylamino group, substituted $C_1$ to $C_{14}$ alkyloxycarbonyl group, substituted $C_1$ to $C_{14}$ acyl group, or substituted $C_1$ to $C_{14}$ acyloxy group in which the substituent is selected from halogen, $C_1$ to $C_{13}$ alkyl, $C_1$ to $C_{13}$ alkoxy, $C_6$ to $C_{13}$ aryloxy, $C_6$ to $C_{13}$ aryl, $C_1$ to $C_{13}$ acylamino, $C_5$ to $C_8$ cyclic amino, $C_1$ to $C_{13}$ mono or dialkylamino, $C_2$ to $C_{13}$ alkyloxycarbonyl, $C_1$ to $C_{13}$ acyl, $C_1$ to $C_{14}$ acyloxy, hydroxy, amino, nitro, cyano and carboxyl groups, in which the $C_1$ to $C_{13}$ alkyl group, $C_1$ to $C_{13}$ alkoxy group, $C_6$ to $C_{13}$ aryloxy group, $C_6$ to $C_{13}$ aryl group and $C_1$ to $C_{13}$ mono or dialkylamino groups may be substituted with substituents selected from halogen, $C_1$ to $C_{13}$ alkyl, $C_1$ to $C_{13}$ alkoxy, $C_6$ to $C_{13}$ aryloxy, $C_6$ to $C_{13}$ aryl, $C_1$ to $C_{13}$ acylamino, $C_5$ to $C_8$ cyclic amino, $C_1$ to $C_{13}$ mono or dialkylamino, $C_2$ to $C_{13}$ alkyloxycarbonyl, $C_1$ to $C_{13}$ acyl, $C_1$ to $C_{14}$ acyloxy, hydroxy, amino, nitro, cyano and carboxyl groups; $R^{15}$ independently represents a hydrogen atom, halogen atom, unsubstituted $C_1$ to $C_{14}$ alkyl group, unsubstituted $C_1$ to $C_{14}$ alkoxy group, unsubstituted $C_1$ to $C_{14}$ acylamino group, unsubstituted $C_1$ to $C_{14}$ alkylamine group, unsubstituted $C_1$ to $C_{14}$ alkyloxycarbonyl group, unsubstituted $C_1$ to $C_{14}$ acyl group, unsubstituted $C_1$ to $C_{14}$ acyloxy group; p is an integer of 1 to 5; q is an integer of 0 to 4;; and p and q together do not exceed 5; and

m is an integer of 0 to 3, n is 0 or 1, and m and n together do not exceed 3, but when n = 0, $R^8$ represents the group of formula (IX), and when n = 1 the substitution position of X is the 6-position or 7-position of the benzopyran ring.

In accordance with the present invention, there is also provided pharmaceutical compositions including these compounds, as an effective ingredient, and drugs against hyperlipidemia.

BEST MODE FOR CARRYING OUT THE INVENTION

The present inventors engaged in intensive research into benzopyran derivatives and, as a result, discovered novel benzopyran derivatives having a substituted urea group or acylamino group on the aromatic ring thereof and, more suprisingly, discovered that these novel compounds have an ACAT enzyme inhibitory activity, cause a reduction in the cholesterol in the blood or in the arterial wall, and exhibit a superior therapeutic effect, and thus completed the present invention.

In the above-mentioned formula (I), X independently represents an unsubstituted or substituted $C_7$ to $C_{20}$ alkyl group, an unsubstituted or substituted $C_7$ to $C_{20}$ alkoxy group, an substituted or $C_7$ to $C_{20}$ acylamino group, an unsubstituted or substituted $C_{13}$ to $C_{30}$ alkylamino group, an unsubstituted or substituted $C_8$ to $C_{20}$ alkyloxcarbonyl group, an unsubstituted or substituted $C_8$ to $C_{20}$ acyl group, an unsubstituted or substituted $C_{13}$ to $C_{20}$ acyloxy group, an unsubstituted or substituted $C_6$ to $C_{20}$ aryl group, or an unsubstituted or substituted $C_6$ to $C_{20}$ aryloxy group.

As the $C_7$ to $C_{20}$ alkyl group, there may be mentioned a $C_7$ to $C_{20}$ chain (straight or branched) or cyclic alkyl group (hereinafter, unless specified to the contrary in the present invention, an alkyl group is considered to include a straight or branched chain or cyclic alkyl group), for example, a heptyl, decyl, eicosyl, 2,2-dimethyloctyl, cyclohexylbutyl group, etc.

As the $C_7$ to $C_{20}$ alkoxy group, there may be mentioned an alkoxy group comprising a $C_7$ to $C_{20}$ alkyl group and an oxy group, for example, a heptyloxy, decyloxy, eicosyloxy, 2-methyl-decyloxy, cyclopetylpropyloxy group, etc.

As the $C_7$ to $C_{20}$ acylamino group, there may be mentioned an acylamino group comprising a $C_7$ to $C_{20}$ acyl group and an amino group, for example, an octanoylamino, decanoylamino, 3, 3-diethylpropanoylamino group, etc.

As the $C_{13}$ to $C_{30}$ alkylamino group, there may be mentioned a mono or dialkylamino group comprising the same or different $C_1$ to $C_{15}$ alkyl groups, for example, octylamino, N-decyl-N-methylamino, didodecylamino group, etc.

As the $C_8$ to $C_{20}$ alkyloxcarbonyl group, there may be mentioned an alkyloxcarbonyl group comprising a $C_8$ to $C_{20}$ alkoxy group and a carbonyl group, for example, a heptyloxycarbonyl, decyloxycarbonyl, 2-ethyl-4-butyldodecyloxcarbonyl group, etc.

As the $C_8$ to $C_{20}$ acyl group, there may be mentioned an acyl group comprising a $C_7$ to $C_{19}$ alkyl group and a carbonyl group, for example, an octanoyl, pentadecanoyl, 3,3-diethylhexanoyl group, etc.

As the $C_{13}$ to $C_{20}$ acyloxy group, there may be mentioned an acyloxy group comprising a $C_{13}$ to $C_{20}$ acyl group and an oxy group, for example, tridecanoyloxy, 2-methylpentadecanoyloxy, cyclopentyldecanoyloxy group, etc.

As the aryl group, there may be mentioned a $C_6$ to $C_{20}$ aryl group, for example, phenyl, 2,4, 6-trimethylphenyl, 4-decylphenyl, etc.

As the $C_6$ to $C_{20}$ aryloxy group, there may be mentioned an aryloxy group comprising a $C_6$ to $C_{20}$ aryl group and an oxy group, for example, a phenyloxy, 2,4,6-trimethylphenyloxy, 4-decylphenyloxy group, etc.

As the substituent groups, when X is substituted alkyl group, alkoxy group, acylamino group, alkylamino group, alkyloxycarbonyl group, acyl group, acyloxyl group, aryl group, and aryloxy group, the substituent group is selected from a halogen atom, hydroxyl group, amino group, alkoxy group and aryloxy group. For example, as the substituted alkyl group, there may be mentioned the methoxydecyl group etc.

Among these, as X, an unsubstituted or substituted $C_7$ to $C_{20}$ alkoxy group or unsubstituted or substituted $C_{13}$ to $C_{30}$ alkylamino group is preferred.

$R^1$ independently represents a hydrogen atom, halogen atom, unsubstituted or substituted $C_1$ to $C_6$ lower alkyl group, unsubstituted or substituted $C_1$ to $C_6$ lower alkoxy group, hydroxyl group, nitro group, cyano group, unsubstituted or substituted $C_1$ to $C_6$ lower acylamino group, unsubstituted or substituted $C_1$ to $C_{12}$ alkylamino group, unsubstituted or substituted $C_1$ to $C_7$ lower alkyloxycarbonyl group, carboxyl group, unsubstituted or substituted $C_1$ to $C_7$ lower acyl group, or unsubstituted or substituted $C_1$ to $C_{12}$ acyloxy group.

As the halogen atom, there may be mentioned, for example, a fluorine atom, chlorine atom, bromine atom, etc.

As the $C_1$ to $C_6$ lower alkyl group, there may be mentioned a chain (straight or branched) or cyclic lower alkyl group (hereinafter, unless specified to the contrary in the present invention, a lower alkyl group is considered to include a straight or branched chain or cyclic alkyl group), for example, a methyl, ethyl, isopropyl, tert-butyl, sec-butyl, pentyl, hexyl, cyclopropyl, cyclohexyl group, etc.

As the $C_1$ to $C_6$ lower alkoxy group, there may be mentioned an alkoxy group comprising a $C_1$ to $C_6$ lower alkyl group and an oxy group, for example, a methoxy, ethoxy, propoxy, tert-butyloxy group, etc.

As the $C_1$ to $C_6$ lower acylamino group, there may be mentioned an acylamino group comprising a $C_1$ to $C_6$ lower acyl group and an amino group, for example, an acetylamino, propanoylamino, butanoylamino group, etc.

As the $C_1$ to $C_{12}$ alkylamino group, there may be mentioned a mono- or di-alkylamino group comprising the same or different $C_1$ to $C_{12}$ alkyl group and amino group, for example, methylamino, ethylamino, dimethylamino, diethylamino,N-butyl-N-methylamino group, etc.

As the $C_1$ to $C_6$ lower alkyloxcarbonyl group, there may be mentioned an alkyloxycarbonyl group comprising a $C_1$ to $C_6$ alkoxy group and a carbonyl group, for example, a methoxycarbonyl, ethoxycarbonyl, propoxycarbonyl, tert-butyloxycarbonyl group etc.

As the $C_1$ to $C_7$ lower acyl group, there may be mentioned an acyl group comprising a $C_1$ to $C_6$ alkyl group and a carbonyl group, for example, an acetyl, propanoyl, butanoyl, benzoyl group, etc.

As the $C_1$ to $C_{12}$ acyloxy group, there may be mentioned an acyloxy group comprising a $C_1$ to $C_{12}$ acyl group and an oxy group, for example, an acetoxy, propanoyloxy, butanoyloxy, benzoyloxy group, etc.

As the substituent groups, when $R^1$ is substituted lower akyl group, lower alkoxy group, or lower alkyloxycarbonyl group, etc., the substituent group is selected from a halogen atom, hydroxy group and amino group. For example, as such a substituted lower alkyl group etc., there may be mentioned, for example, a trifluoromethyl group etc. Among these, as $R^1$, a hydrogen atom, halogen atom, unsubstituted or substituted lower alky group, or unsubstituted or substituted lower alkoxy group is preferable.

$R^2$ and $R^3$ are combined together to form an oxygen atom, $R^4$ and $R^6$ may be combined together to form a carbon-carbon bond but otherwise $R^4$ to $R^7$ each represent a hydrogen atom.

Further, in the formula (I), $R^8$ represents a $C_5$ to $C_{20}$ cycloalkyl or cycloalkenyl group or an unsubstituted or substituted $C_6$ to $C_{20}$ aryl group, or a group of the formula (VII):

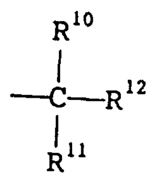

(VII)

As the substituted $C_6$ to $C_{20}$ aryl group, there may be mentioned an aromatic hydrocarbon group substituted with a halogen atom, $C_1$ to $C_{14}$ alkyl group, alkoxy group, acylamino group, mono- or di-alkylamino group, alkyloxycarbonyl group, acyl group, and acyloxy group. These substituent groups may further be substituted with a halogen atom, hydroxyl group, nitro group, cyano group, $C_1$ to $C_{10}$ alkoxy group, $C_6$ to $C_{10}$ aryloxy group, or $C_1$ to $C_{12}$ alkylamino group.

Among these, as the $R^8$, there may preferably be mentioned a p-fluorophenyl, p-decylphenyl, p-methoxyphenyl, p-decyloxyphenyl, p-decylaminophenyl, p-decylmethylaminophenyl, 6-(p-chlorophenoxy)-hexyloxyphenyl, 6-butyloxy-hexyloxyphenyl, etc.

$R^{10}$ and $R^{11}$ independently represent a hydrogen atom or $C_1$ to $C_6$ lower alkyl group.

As the $C_1$ to $C_6$ lower alkyl group, there may be mentioned, for example, a methyl, ethyl, propyl, isopropyl, pentyl, neopentyl, hexyl, cyclohexyl, cyclopropylmethyl group, etc.

$R^{12}$ represents a hydrogen atom, unsubstituted or substituted $C_1$ to $C_{19}$ alkyl or alkenyl group, unsubstituted or substituted $C_6$ to $C_{19}$ aryl group, unsubstituted or substituted $C_6$ to $C_{19}$ aryl alkyl group. As specific suitable examples

of the $R^8$ when $R^{12}$ is a $C_1$ to $C_{19}$ alkyl group, there may be mentioned ethyl, propyl, isobutyl, hexyl, isohexyl, octyl, undecyl, dodecyl, tridecyl, pentadecyl, hexadecyl, heptadecyl, nondecyl, eicosyl, 1,1-dimethylheptyl, 1,1-dimethylundecyl, 1,1,12,12-tetramethyltridecyl, 1-methyltridecyl, 1-decylcyclohexyl, 1-decylcyclopentyl, 1-dodecylcyclopropyl, 1-cyclohexyl-1-methylethyl, and 1-ethyloctyl, etc. As suitable specific examples of $R^8$ when $R^{12}$ is a $C_2$ to $C_{12}$ alkenyl group, there may be mentioned propenyl, isopentenyl, hexenyl, 8-tridecenyl, 8-heptadecenyl, 9-octadecenyl, 8,11-heptadecadienyl, 1,1-dimethyl-8-noneyl, cyclohexenylmethyl, etc. As suitable specific examples of $R^8$ when $R^{12}$ is a $C_6$ to $C_{19}$ aryl group, there may be mentioned benzyl, 1-phenylcyclopentyl, 1-phenylethyl, and 1-methyl-1-(2-pyridyl)ethyl, etc. As specific examples of $R^8$ when $R^{12}$ is a $C_6$ to $C_{19}$ aryl alkyl group, there may be mentioned an aryl alkyl group comprising an aromatic hydrocarbon and an alkyl group, an aryl alkyl group comprising an aromatic cyclic compound containing a hetero atom and an alkyl group, for example, 2-phenylethyl, 8-phenyloctyl, 1,1-dimethyl-11-phenylundecyl, 1-benzylcyclopentyl, (1-phenylcyclopentyl)methyl, 1,1-dimethyl-4-(1-methyl-1,2,3,4-tetrahydroquinoline-6-yl)butyl, 1,1-dimethyl-7-pyridylheptyl, 2,2-diphenylethyl, etc. Further, $R^{12}$ may be a group of formula -A-Z-B (wherein, A represents a $C_1$ to $C_{12}$ alkyl group, Z represents an oxygen atom or a sulfur atom, and B represents an unsubstituted or substituted $C_1$ to $C_{19}$ alkyl group, unsubstituted or substituted $C_6$ to $C_{19}$ aryl group, or unsubstituted or substituted $C_6$ to $C_{19}$ aryl alkyl group). The alkyl group in B has the same definition as the lower alkyl group in $R^1$ or the alkyl group in $R^{12}$ and the same may be mentioned as suitable specific examples.

The aryl group and aryl alkyl group in B have the same definitions as the aryl group and aryl alkyl group in $R^{12}$ and the same may be mentioned as suitable specific examples. As suitable specific examples of $R^8$ when $R^{12}$ is a group of formula -A-Z-B, there may be mentioned 6-isobutoxyhexyl, 6-p-chlorophenoxyhexyl, 5-p-dimethylaminophenoxypentyl, 5-isohexyloxy-1,1-dimethylpentyl, 7-isohexyloxy-1,1-dimethylheptyl, 7-isobutoxyl- 1,-1 -dimethylheptyl, 7-neopentyloxy-1,1-dimethylheptyl, 5-p-chlorophenoxy-1,-1-dimethylpentyl, 6-p-chlorophenoxy-1,1-dimethylhexyl, 7-p-chlorophenoxy-1,-1 -dimethylheptyl, 1,1-dimethyl-7-p-tolyloxyheptyl, 5-(p-tert-butylphenoxypentyl), 1,1-dimethyl-6-p-dimethyl-aminophenoxyhexyl, 1,1-dimethyl-7-p-dimethylamino-phenoxyheptyl, 7-isopropylamino-1,1-dimethylheptyl, 7-benzylamino-1,1-dimethylheptyl, 7-(N-benzyl-N-methylamino)-1,1-dimethylheptyl, 7-(N-p-chlorobenzyl-N-methylamino)-1,1-dimethylheptyl, 7-(N-p-chlorophenyl-N-methylamino)-1,1-dimethylheptyl, 1,1-dimethyl-7-piperidinoheptyl, 1,1 -dimethyl-7-(4-methyl-1-piperadinyl)heptyl, 7-(4-benzyl-1-piperadinyl)-1,1-dimethylheptyl, 5-(4-benzyl-1-piperadinyl)- 1,-1-dimethylpentyl, 6-(p-chlorophenylthio)-1,1-dimethylhexyl, etc. Further, the groups of $R^{12}$ may be substituted by one or more of halogen atoms, lower alkyl groups, lower alkoxy groups, hydroxyl groups, nitro groups, cyano groups, lower acylamino groups, lower alkylamino groups, lower alkyloxycarbonyl groups, carboxyl groups, lower acyl groups, or lower acyloxycarbonyl groups. The definitions of these substituent groups are the same as the definitions in $R^1$ and the same suitable specific examples may be mentioned. As suitable specific examples of the $R^8$ including the $R^{12}$ substituted by these substituent groups, there may be mentioned 10-ethoxycarbonyldecyl, 2-(p-nitrophenyl)ethyl, 7-p-chlorophenyl-1,1 -dimethylheptyl, 4-p-isobutylphenyl-1,1-dimethylbutyl, 1-(3-benzoylpropyl)cyclopentyl, 4-p-diethylaminophenyl-1,1-dimethylbutyl, 6-p-ethoxyphenyl-1,1-dimethylhexyl, (1-m-cyanophenylcyclopentyl)methyl, (1-p-dimethylaminophenylcyclopentyl)methyl, (1-p-methoxyphenylcyclopentyl)methyl, etc.

Further, $R^8$ in the above formula (I) represents the group of the formula (IX):

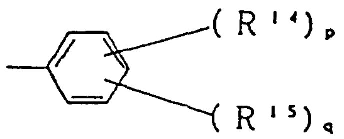

(IX)

$R^{14}$ independently represents a substituted $C_1$ to $C_{14}$ alkyl group, substituted $C_1$ to $C_{14}$ alkoxy group, substituted $C_1$ to $C_{14}$ acylamino group, substituted $C_1$ to $C_{14}$ alkylamino group, substituted $C_1$ to $C_{14}$ alkyloxycarbonyl group, substituted $C_1$ to $C_{14}$ acyl group, or substituted $C_1$ to $C_{14}$ acyloxy group.

These alkyl groups, alkoxy groups, acylamino groups, alkylamino groups, alkyloxycarbonyl groups, acyl groups, and acyloxy groups have the same definitions as the groups in the above $R^1$, except for including greater numbers of carbon atoms, i.e., $C_1$ to $C_{14}$. The definitions of these groups in the following $R^{15}$ are also the same.

As the substituent groups of the substituted $C_1$ to $C_{14}$ alkyl groups etc., there may be mentioned halogen atoms, $C_1$ to $C_{13}$ alkyl groups, $C_1$ to $C_{13}$ alkoxy groups, $C_6$ to $C_{13}$ aryloxy groups, $C_6$ to $C_{13}$ aryl groups, $C_1$ to $C_{13}$ acylamino groups, $C_5$ to $C_8$ cyclic amino groups, $C_1$ to $C_{13}$ mono or dialkylamino groups, $C_2$ to $C_{13}$ alkyloxycarbonyl groups, $C_1$ to $C_{13}$ acyl groups, $C_1$ to $C_{14}$ acyloxyl groups, hydroxyl groups, amino groups, nitro groups, cyano groups, and carboxyl groups. Further, these substituent groups of the $C_1$ to $C_{13}$ alkyl groups may be repeatedly substituted with the here-mentioned substituent groups.

As the substituted $C_1$ to $C_{14}$ alkyl group, there may be mentioned, for example, a trifluoromethyl, 6-methoxyhexyl, 4-chlorophenoxymethyl, 2-(4-methoxymethoxyphenyl)ethyl group, etc.

As the substituted $C_1$ to $C_{14}$ alkoxy group, there may be mentioned, for example, a chloromethoxy, 6-methoxyhexyloxy, 6-(4-chlorophenoxy)hexyloxy, 2-(4-methoxymethoxyphenyl)ethoxy, 2-piperidinoethoxy, 2-morpholinoethoxy group, etc.

As the substituted $C_1$ to $C_{14}$ acylamino group, there may be mentioned, for example, a chloroacetylamino, 6-methoxyhexanoylamino, 4-chlorobenzoylamino, 4-methoxyphenylacetylamino group, etc.

As the substituted $C_1$ to $C_{14}$ alkylamino group, there may be mentioned, for example, a mono- or di-alkylamino group comprising the same or different $C_1$ to $C_{14}$ substituted alkyl groups, for example, a chloromethylamino, 4-chlorobenzylmethylamino, di(6-methoxyhexyl)amino, di(4-t-butyloxyphenylethyl)amino group, etc.

As the substituted $C_1$ to $C_{14}$ alkyloxycarbonyl group, there may be mentioned, for example, a chloromethoxycarbonyl, 6-methoxyhexyloxycarbonyl, 6-(4-chlorophenoxy) hexyloxycarbonyl, 2-(4-methoxymethoxyphenyl)ethoxycarbonyl group, etc.

As the substituted $C_1$ to $C_{14}$ acyl group, there may be mentioned, for example, a chloroacetyl, 6-methoxyhexanoyl, 4-chlorobenzoyl, 4-methoxyphenylacetyl group, etc.

As the substituted $C_1$ to $C_{14}$ acyloxyl group, there may be mentioned, for example, a chloroacetyloxy, 6-methoxyhexanoyloxy, 4-chlorobenzoyloxy, 4-methoxyphenylacetyloxy group, etc. Among these, the substituted $C_1$ to $C_{14}$ alkoxy groups or the substituted $C_1$ to $C_{14}$ alkylamino groups are preferable as the $R^{14}$.

$R^{15}$ independently represents a hydrogen atom, halogen atom, unsubstituted $C_1$ to $C_{14}$ alkyl group, unsubstituted $C_1$ to $C_{14}$ alkoxy group, unsubstituted $C_1$ to $C_{14}$ acylamino group, unsubstituted $C_1$ to $C_{14}$ alkylamino, unsubstituted $C_1$ to $C_{14}$ alkyloxycarbonyl group, unsubstituted $C_1$ to $C_{14}$ acyl group, or unsubstituted $C_1$ to $C_{14}$ acyloxyl group. As the halogen atom, there may be mentioned, for example, a fluorine atom, chlorine atom, bromine atom.

As the unsubstituted $C_1$ to $C_{14}$ alkyl group, there may be mentioned, for example, a methyl, ethyl, isopropyl, cyclohexyl, decyl, tetradecyl group, etc.

As the unsubstituted $C_1$ to $C_{14}$ alkoxy group, there may be mentioned, for example, a methoxy, ethoxy, isopropoxy, cyclohexyloxy, decyloxy, tetradecyloxy group, etc.

As the unsubstituted $C_1$ to $C_{14}$ acylamino group, there may be mentioned, for example, an acetylamino, propanoylamino, isobutanoylamino, cyclohexylcarbonylamino, decanoylamino, tetradecanoylamino group, etc.

As the unsubstituted $C_1$ to $C_{14}$ alkylamino group, there may be mentioned a mono- or di-alkylaminogroup comprising the same or different unsubstituted $C_1$ to $C_{14}$ alkyl group, for example, a methylamino, ethylamino, diisopropylamino, methylcyclohexylamino, decylaminogroup, etc.

As the unsubstituted $C_1$ to $C_{14}$ alkyloxycarbonyl group, there may be mentioned a methoxycarbonyl, ethoxycarbonyl, isopropoxycarbonyl, cyclohexyloxycarbonyl, decyloxycarbonyl, tetradecyloxycarbonyl group, etc.

As the unsubstituted $C_1$ to $C_{14}$ acyl group, there may be mentioned, for example, an acetyl, propanoyl, isobutanoyl, decanoyl, cyclohexylacetyl group, etc.

As the unsubstituted $C_1$ to $C_{14}$ acyloxy group, there may be mentioned, for example, an acetyloxy, propanoyloxy, isobutanoyloxy, decanoyloxy, cyclohexylacetyloxy group, etc.

Among these, a hydrogen atom, halogen atom, unsubstituted $C_1$ to $C_{14}$ alkyl group, or unsubstituted alkoxy group is preferred as the $R^{15}$.

p is an interger of 1 to 5; q is an integer of 0 to 4; and p and q together do not exceed 5.

In the above formula (I), m is an integer of 0 to 3, and n is an integer of 0 or 1. Further, m and n together do not exceed 3.

According to the present invention, the benzopyran derivatives of the present invention may have a basic group in their molecules. At that time, they may be acid adducts. As the acid, there may be mentioned, for example, mineral acids such as hydrochloric acid, hydrobromic acid, sulfuric acid, phosphoric acid, and carbonic acid, and organic acids such as, citric acid, malic acid, oxalic acid, tartaric acid, fumaric acid, and methanesulfonic acid, etc.

According to the present invention, the following compounds may be mentioned as suitable specific examples of the benzopyran derivatives of the formula (I) Further, the compounds of the present invention include all optical isomers when having an asymmetric carbon atom in the structural formula:

As suitable specific examples when n is not 0, there are

(101) N-(7-decyloxy-4-chromanon-8-yl)-2, 2-dimethylpropanamide (see Example 1)
(102) N-(7-decyloxy-4-chromanon-8-yl)-4-chlorobenzamide (see Example 2)
(103) N-(7-decyloxy-4-chromanon-8-yl)acetamide (see Example 3)
(104) N-(7-decyloxy-4-chromanon-8-yl)-2, 2-dimethyldodecanamide (see Example 4)
(105) 8-(4-chlorophenoxy)-N-(7-decyloxy-4-chromanon-8-yl)-2,2-dimethyloctanamide (see Example 5)
(106) 4-decyloxy-N-(7-decyloxy-4-chromanon-8-yl)benzamide (see Example 6)
(107) 4-(6-(4-chlorophenoxy)hexyloxy)-N-(7-decyloxy-4-chromanon-8-yl)benzamide (see Example 7)
(108) N-(7-decyloxy-4-oxo-4H-1-benzopyran-8-yl)-2, 2-dimethylpropanamide (see Example 8)

(109) N-(7-(4-methoxyphenyl)butoxy)-4-chromanon-8-yl)-2, 2-dimethylpropanamide (see Example 9)
(110) N-(7-(6-phenylhexyloxy)-4-chromanon-8-yl)-2, 2-dimethylpropanamide (see Example 10).

(111)

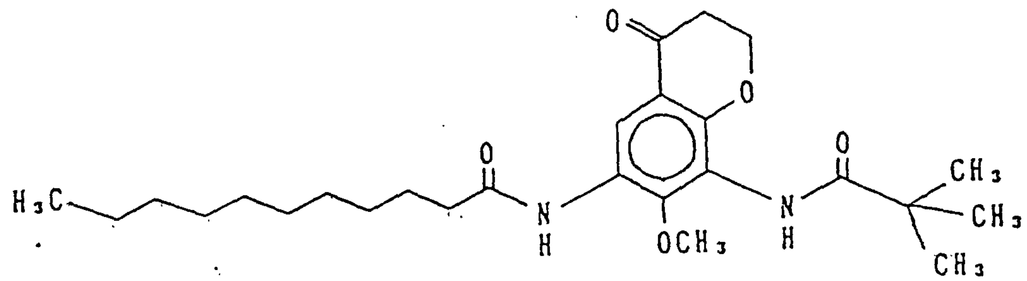

(112)

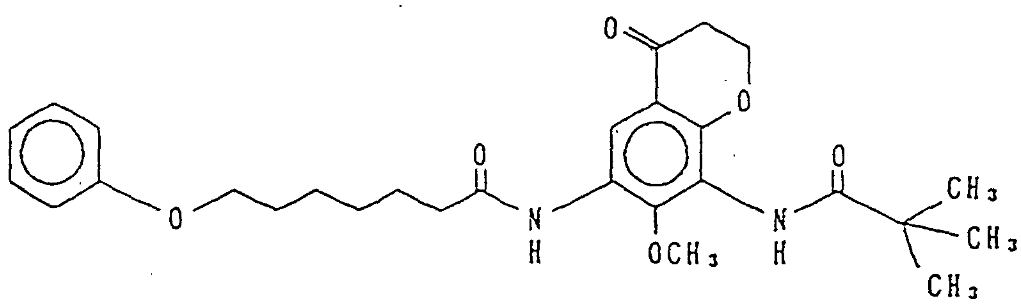

(113)

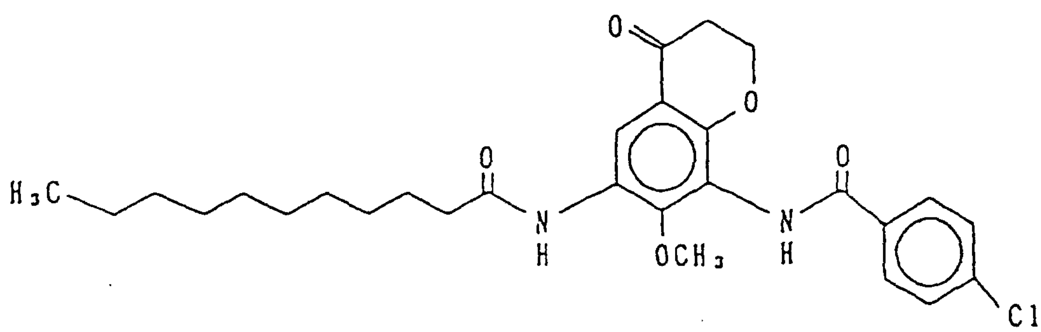

(114)

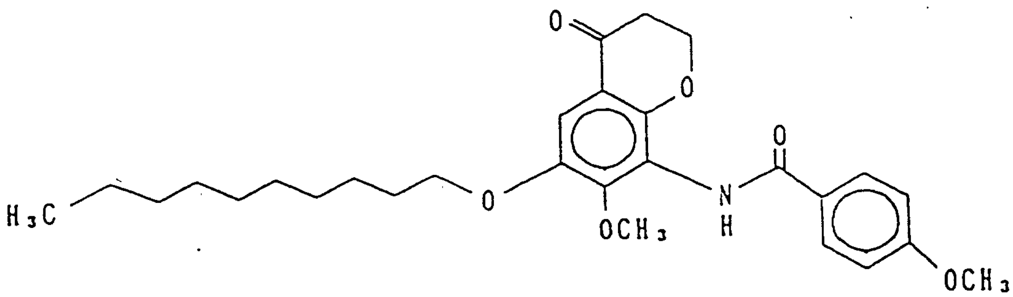

(116)

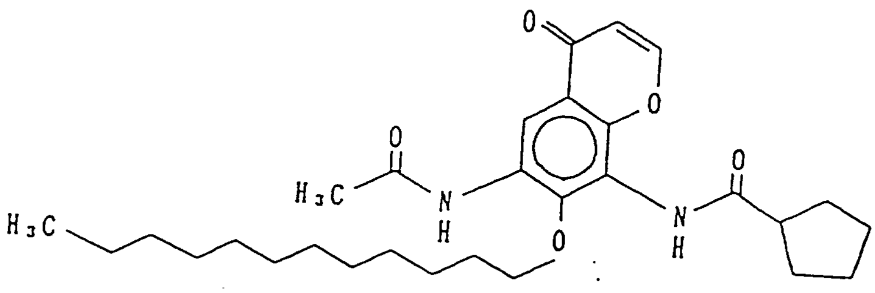

(119)

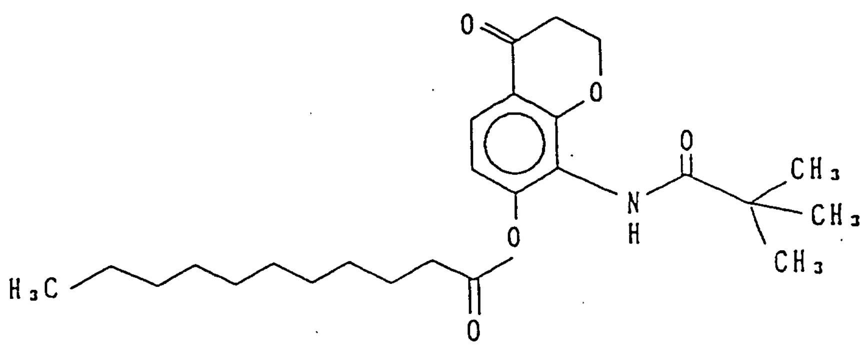

(121)

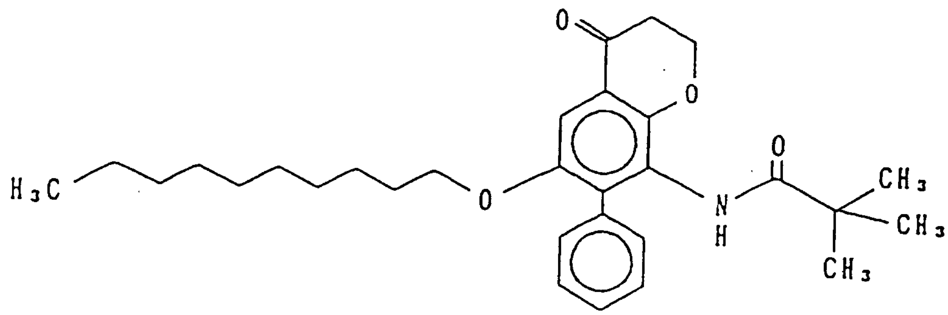

(122)

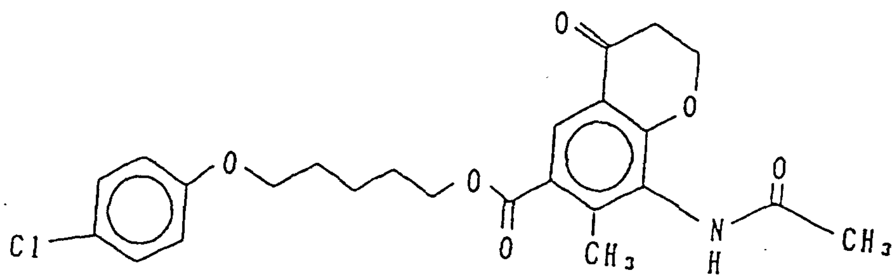

In the above formula (I), as suitable specific examples of when n is 0 or 1 to 3 and $R^8$ is the formula (IX), there may be mentioned:

(201) N-(7-methoxy-4-chromanon-8-yl)-4-(6-(4-chlorophenoxy)hexyloxy)benzamide (see Example 11)
(202) N-(7-methoxy-4-chromanon-8-yl)-4-(4-chlorobenzyloxy)benzamide (see Example 12)
(203) N-(7-methoxy-4-chromanon-8-yl)-4-(6-(2-methylpropyloxy)hexyloxy)benzamide (see Example 13)
(204) N-(7-methoxy-4-chromanon-8-yl)-4-(6-propoxyhexyloxy)benzamide (see Example 14)
(205) N-(7-methoxy-4-chromanon-8-yl)-4-(3-dimethylaminopropoxy) benzamide (see Example 15)
(206) N-(7-methoxy-4-chromanon-8-yl)-4-(2-piperidinoethyloxy) bezamide (see Example 16)

(207)

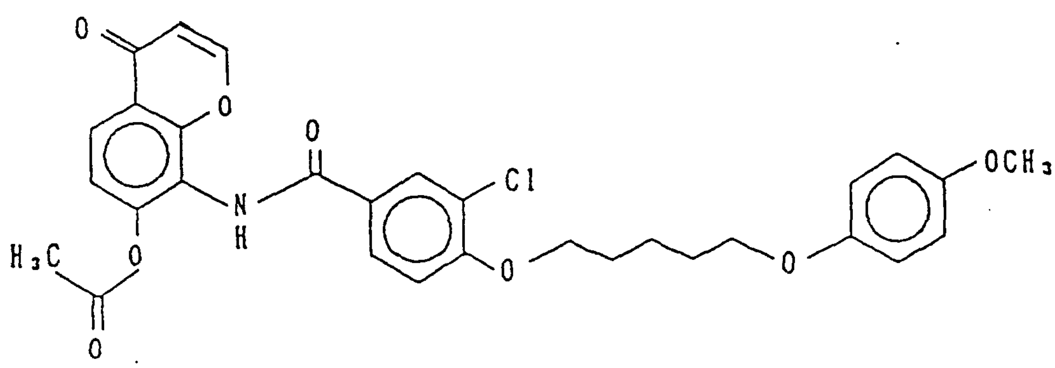

(209)

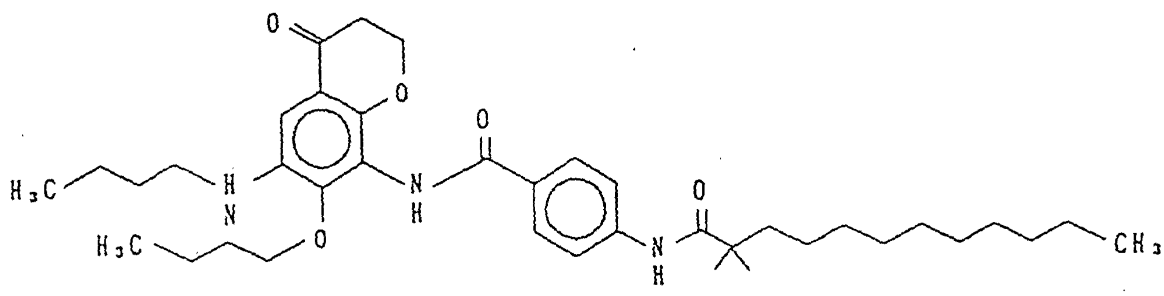

(210)

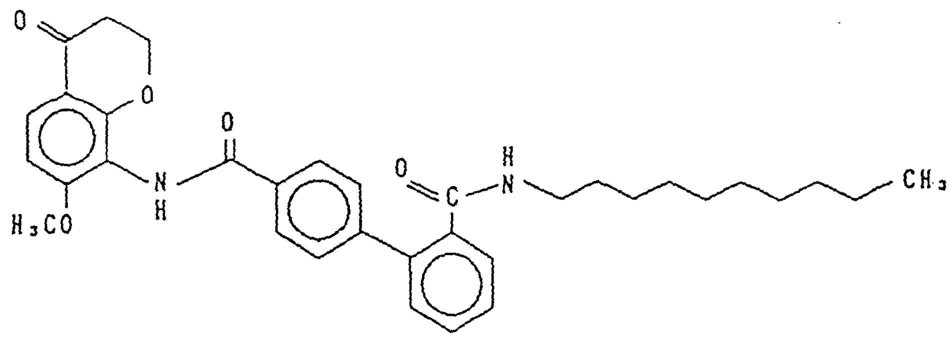

(211)

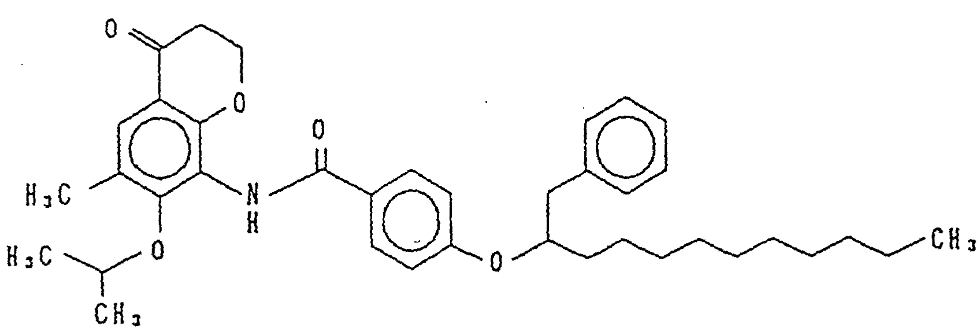

(212)

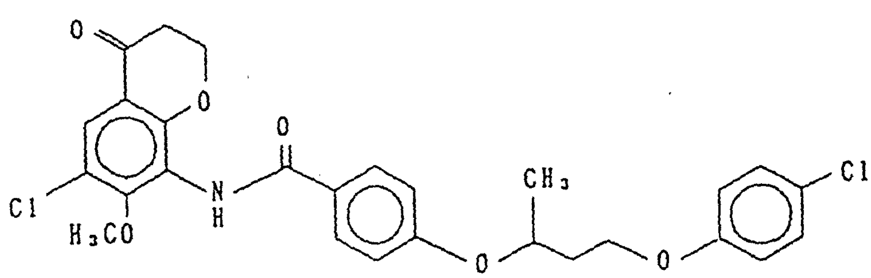

(213)

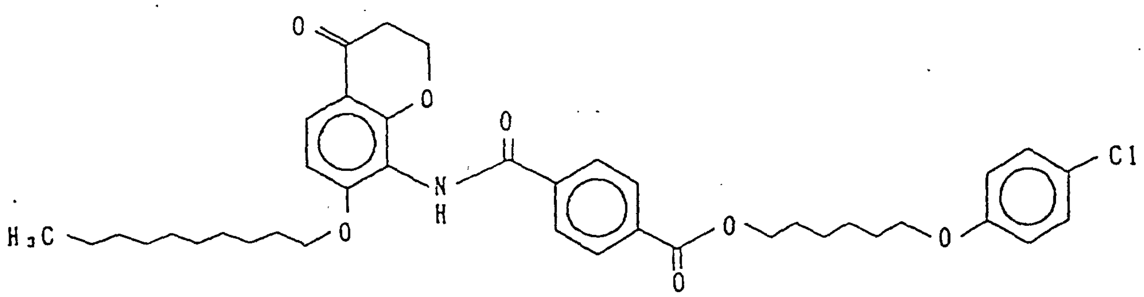

(214)

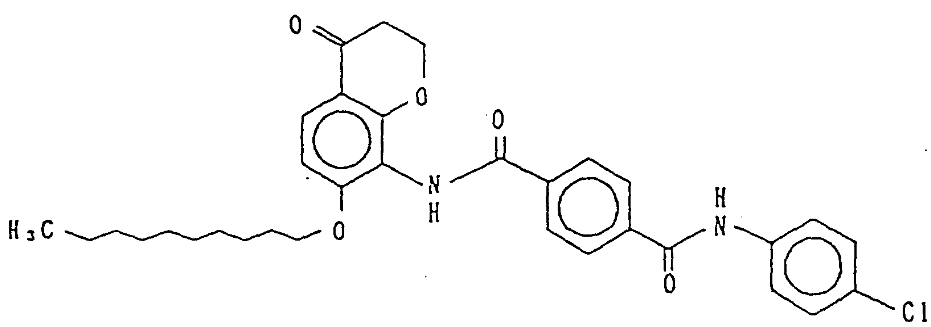

(215)

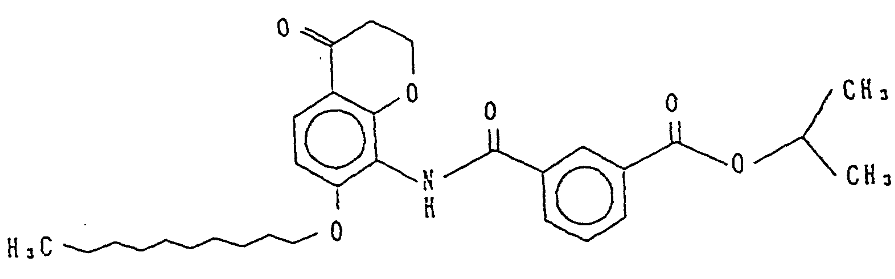

(216)

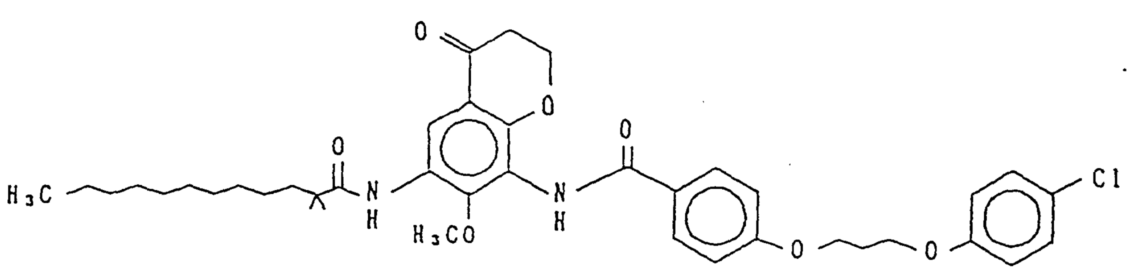

(217)

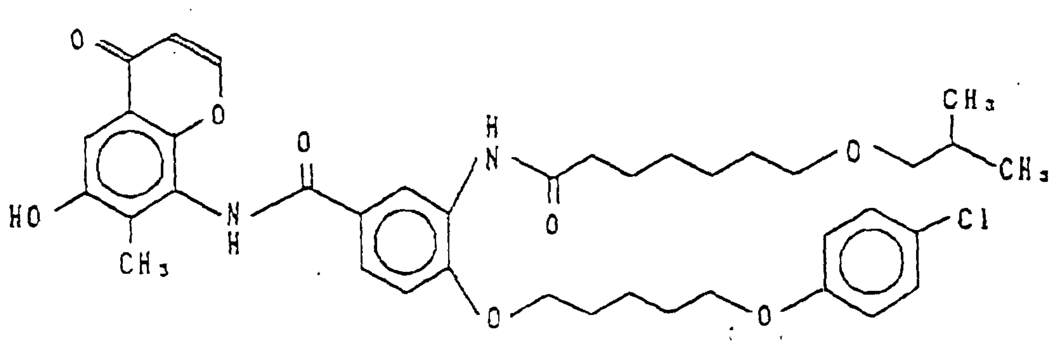

As the compounds of the formula (I) of the present invention, there may be mentioned those having either of the benzopyran rings selected from chromanone derivatives, in which $R^2$ and $R^3$ are combined together to form an oxygen atom and $R^4$ and $R^6$ together to not form a carbon-carbon bond, and 4-oxobenzopyran derivatives, in which $R^2$ and $R^3$ are combined together to form an oxygen atom and $R^4$ and $R^6$ are combined together to form a carbon bond, and the benzopyran rings may have the same substituents with the specific examples (101) to (217). Among these chromanone and 4-oxobenzopyran derivatives, there may be preferably mentioned chromanone derivatives.

Regarding the substituent group of the benzopyran ring in formula (I), there may be preferably mentioned benzopyran derivatives, and pharmacologically acceptable acid adducts thereof, wherein the substitution position of the group Z is the 8-position or 5-position of the benzopyran ring, in particular the 8-position, and furthermore benzopyran derivatives and pharmacologically acceptable acid adducts thereof, wherein the substitution position of the group Z is the 8-position of the benzopyran ring and there is a substituent group other than a hydrogen atom at the 7-position. Further, there may be mentioned benzopyran derivatives and pharmacologically acceptable acid adducts thereof, wherein the substituent group of the 7-position of the benzopyran ring is an unsubstituted or substituted $C_1$ to $C_{20}$ alkyl group or unsubstituted or substituted $C_1$ to $C_{20}$ alkoxy group, in particular, benzopyran derivatives and pharmacologically acceptable acid adducts thereof, wherein the substituent group at the 7-position of the benzopyran ring is an unsubstituted or substituted $C_1$ to $C_{20}$ alkoxy group or the substituent group of the 7-position of the benzopyran ring is the group X and the group Z has seven carbon atoms or less.

Futhermore, benzopyran derivatives in which, in the formula (I), n is 0 and Z is a group of the formula (IX), in particular, benzopyran derivatives and pharmacologically acceptable acid adducts thereof, in which the group Z is located at the 8-position of the benzopyran ring and there is a substituent group other than a hydrogen atom at the 7-position are preferable compounds.

As the benzopyran rings in the case where the substituent groups of the benzopyran ring in the compounds of the formula (I) of the present invention are the preferable groups mentioned above, there may be preferably mentioned the above-mentioned chromanones and 4-oxobenzopyran derivatives, in particular, chromanone derivatives.

In accordance with the present invention, it is further possible to provide a process for producing benzopyran derivatives having acylamino groups, and the pharmacologically acceptable acid adducts thereof, by allowing to react between a 1-benzopyranylamine of the formula (IV):

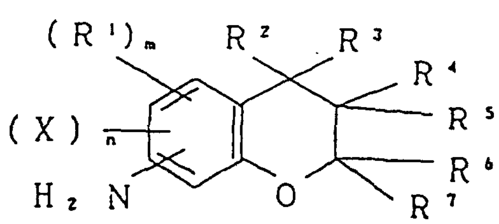

··· (IV)

(wherein, $R^1$ to $R^7$, x, m, and n have the same definitions as the $R^1$ to $R^7$, x, m, and n in the above formula (I)) and a carboxylic acid, or their reactive derivatives, of the following formula (VI):

$$R^8\text{-COOH} \qquad (VI)$$

(wherein $R^8$ has the same definition as the $R^8$ in the formula (I)). The 1-benzopyranylamine of the formula (IV) may be produced in accordance with conventional known processes.

The carboxylic acid, or their reactive derivatives, of the above formula (VI) may be produced in accordance with conventionally known methods.

The production process provided by the present invention may be carried out by, for example, allowing to react between one equivalent of 1-benzopyranylamine of the above formula (IV) and 1 to 5 equivalents of carboxylic acid of the formula (VI) with a condensing agent, for example, 1 to 2 equivalents of dicyclohexylcarbodiimide and 0.1 to 1.5 equivalents of a basic amine, for example, triethylamine, pyridine, and 4-dimethylaminopyridine, in the presence of a solvent.

The reaction temperature is -20 to 150°C, preferably room temperature to 100°C. The reaction time is usually within 72 hours.

Alternatively, it is possible to allow to react between one equivalent of 1-benzopyranylamine of the above formula (IV) and 1 to 5 equivalents of a reactive derivative of the carboxylic acid of the formula (VI), for example, the corresponding acid chloride or acid anhydride, in the presence of a solvent.

The reaction temperatures is -20 to 150°C, preferably -10°C to 100°C. The reaction time is usually within 72 hours.

As the solvent, for example, use may be made of halogenated hydrocarbons such as dichloromethane, chloroform, aromatic hydrocarbons such as bezene, toluene, ethers such as diethyl ether, tetrahydrofuran, and aprotic polar solvents such as dimethylformamide, dimethylsulfoxide. In this case, 0.1 to 1.5 equivalents of basic amines, for example, triethylamine, pyridine, 4-dimethylaminopyridine, etc. may be added.

After the end of the reaction, the usual separation and purification operations, that is, extraction, recrystallisation, chromatography, etc. may be performed so as to isolate the desired benzopyran derivatives of the formula (I). Further, the usual methods may be used to convert them into their pharmacologically acceptable acid adducts.

The benzopyran derivatives and their pharmacologically acceptable acid adducts provided by the present invention have ACAT enzyme inhibitory activity and have superior pharmacological action in reducing the total cholesterol and LDL in the blood, liver, and arterial walls. Therefore, they are useful for the suppression and regression of atherosclerosis and hyperlipidemia.

The benzopyran derivatives and their pharmacologically acceptable acid adducts provided by the present invention may be administered orally or nonorally.

As the forms of the oral preparations, for example, there may be mentioned tablets, powder, granules, capsules, etc. To form the preparations, for example, it is possible to use usual methods using lactose, starch, crystal cellulose, and other excipients, carboxymethylcellulose, methylcellulose, polyvinylpyrrolidone, and other binders, and sodium alginate, sodium hydrogen carbonate, sodium laurate, and other disintegrants. The powder and granules may be formed by similar methods. The capsules are formed by filling powder, granules, etc. in gelatin and other capsules.

As the parenteral agents, for example, there may be mentioned suppositories, patches and other percutaneous agents, injections, etc.

The dosage of the benzopyran derivatives and their pharmacologically acceptable acid adducts according to the present invention differs depending on the severity of the illness, age and sex of the patient, etc., but for usual adults is 1 to 500 mg/day.

## EXAMPLES

The present invention will now be explained in further detail by, but is by no means limited to, the following Examples.

Example 1

Preparation of N-(7-decyloxy-4-chromanon-8-yl)-2,2-dimethylpropanamide (Compound 101)

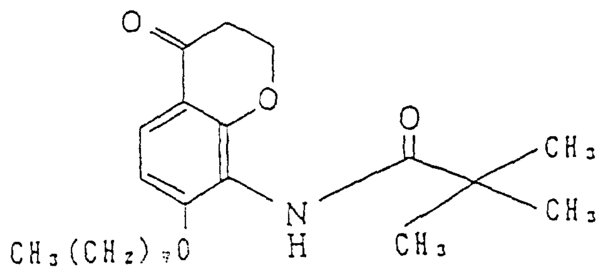

A 1.14 mL amount of triethylamine was added to a solution of 2.17 g of 8-amino-7-decyloxy-4-chromanon in 20 mL of 1,2-dichloroethane. A 0.84 mL amount of trimethylacetyl chloride was added in small portions. The mixture was stirred at room temperature for 20 hours.

After the reaction, extraction was performed by dichloromethane. Drying was performed, then the solvent was distilled off under reduced pressure. Recrystallization from a mixed solvent of hexane and ethyl acetate gave 2.09 g of the desired above-identified compound.

$^1$H-NMR ($CDCl_3$) δ/ppm

7.82 (d, J=8.9Hz, 1H), 6.79 (s, 1H), 6.61 (d, J=8.9Hz, 1H), 4.54 (t, J=6.6Hz, 2H), 4.03 (t, J=6.6Hz, 2H), 2.77 (t, J=6.6Hz, 2H), 1.77 (tt, J=6.6Hz, 2H), 1.26-1.43 (m, 14H), 1.34 (s, 9H), 0.88 (t, J=6.6Hz, 3H)

Melting point: 156-158°C

Example 2

Preparation of N-(7-decyloxy-4-chromanon-8-yl)-4-chlorobenzamide (Compound 102)

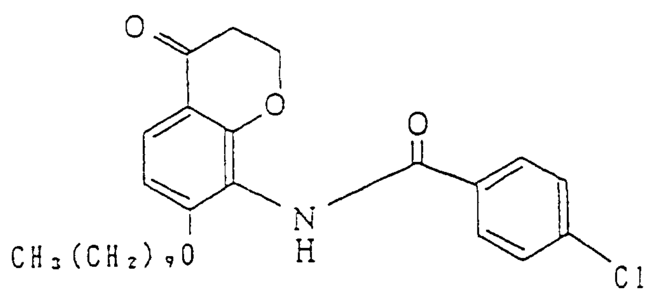

The same procedure was performed as in Example 1, except that use was made of 51.4 mg of 8-amino-7-decyloxy-4-chromanone, to obtain 50.9 mg of the desired above-identified compound.

$^1$H-NMR ($CDCl_3$) δ/ppm

7.88 (d, J=8.9Hz, 1H), 7.86 (d, J=8.6Hz, 2H), 7.47 (d, J=8.6Hz, 2H), 7.21 (s, 1H), 6.68 (d, J=8.9Hz, 1H), 4.55 (t, J=6.3Hz, 2H), 4.07 (t, J=6.6Hz, 2H), 2.80 (t, J=6.3Hz, 2H), 1.75 (tt, J=6.6Hz, 2H), 1.21-1.37 (m, 14H), 0.88 (t, J=6.6Hz, 3H)

Melting point: 155-157°C

Example 3

Preparation of N-(7-decyloxy-4-chromanon-8-yl)acetamide (Compound 103)

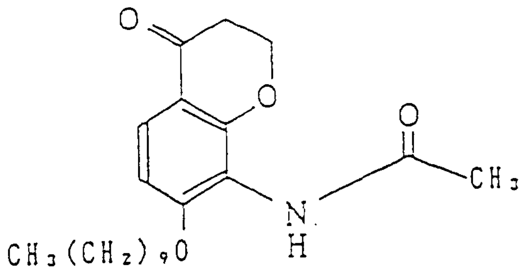

The same procedure was performed as in Example 1, except that use was made of 404 mg of 8-amino-7-decyloxy-4-chromanone, to obtain 338 mg of the desired above-identified compound.

$^1$H-NMR ($CDCl_3$) δ/ppm

7.85 (d, J=8.9Hz, 1H), 6.64 (d, J=8.9Hz, 1H), 4.55 (t, J=6.6Hz, 2H), 4.05 (t, J=6.6Hz, 2H), 2.78 (t, J=6.6Hz, 2H), 2.17 (br, 3H), 1.79 (tt, J=6.6,6.6Hz, 4H), 1.11-1.50 (m, 14H), 0.88 (t, J=6.6Hz, 3H)

IR (KBr)

3246, 2914, 2851, 1680, 1655, 1535, 1445, 1381, 1292 $cm^{-1}$

Melting point: 151.5-152.5°C

Example 4

Preparation of N-(7-decyloxy-4-chromanon-8-yl)-2,2-dimethyldodecanamide (Compound 104)

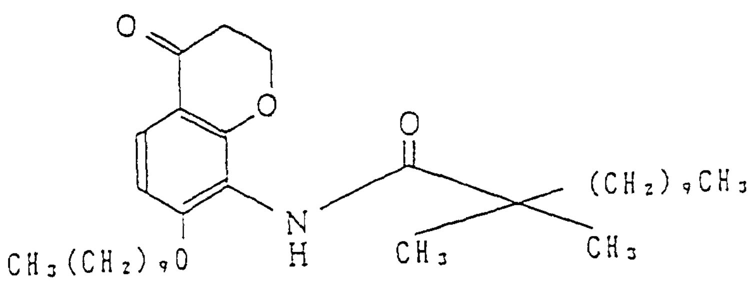

The same procedure was performed as in Example 1, except that use was made of 100 mg of 8-amino-7-decyloxy-4-chromanone, to obtain 154 mg of the desired above-identified compound.

$^1$H-NMR ($CDCl_3$) δ/ppm

7.82 (d, J=9.9Hz, 1H), 6.76 (s, 1H), 6.61 (d, J=9.9Hz, 1H), 4.52 (t, J=6.3Hz, 2H), 4.02 (t, J=6.3Hz, 2H), 2.77 (t, J=6.4Hz, 2H), 1.7-1.8 (m, 2H), 1.0-1.7 (m, 38H), 0.88 (t, J=5.3Hz, 6H)

Example 5

Preparation of 8-(4-chlorophenoxy)-N-(7-decyloxy-4-chromanon-8-yl)-2,2-dimethyloctanamide (Compound 105)

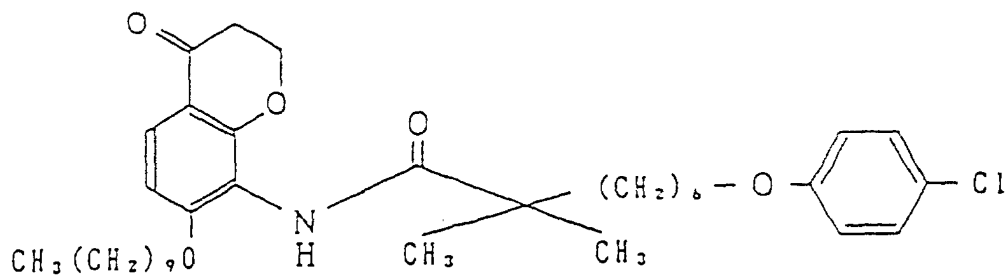

The same procedure was performed as in Example 1, except that use was made of 100 mg of 8-amino-7-decyloxy-4-chromanone, to obtain 178 mg of the desired above-identified compound.

$^1$H-NMR ($CDCl_3$) δ/ppm

7.82 (d, J=9.9Hz, 1H), 7.21 (d, J=9.9Hz, 2H), 6.76 (s, 1H), 6.80 (d, J=9.9Hz, 1H), 6.62 (d, J=9.9Hz, 1H), 4.52 (t, J=6.3Hz, 2H), 4.02 (d, J=6.3Hz, 2H), 3.91 (t, J=6.6Hz, 2H), 2.76 (t, J=6.3Hz, 2H), 1.3-1.9 (m, 32H), 0.88 (t, J=6.4Hz, 3H)

Example 6

Preparation of 4-decyloxy-N-(7-decyloxy-4-chromanon-8-yl)benzamide (Compound 106)

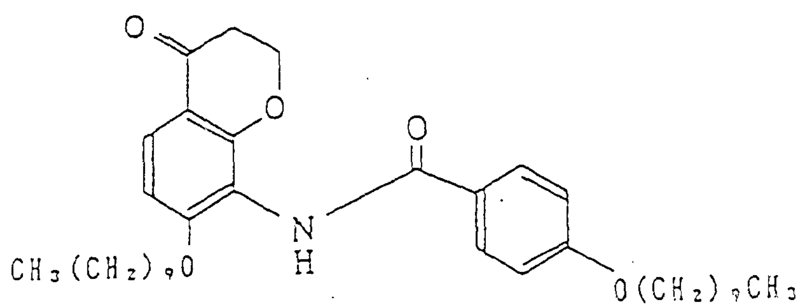

The same procedure was performed as in Example 1, except that use was made of 224 mg of 8-amino-7-decyloxy-4-chromanone, to obtain 349 mg of the desired above-identified compound.

$^1$H-NMR ($CDCl_3$) δ/ppm

7.88 (d, J=8.6Hz, 2H), 7.86 (d, J=8.9Hz, 1H), 6.96 (d, J=8.6Hz, 2H), 6.67 (d, J=8.9Hz, 1H), 4.55 (t, J=6.1Hz, 2H), 4.06 (t, J=6.3Hz, 2H), 4.02 (t, J=6.4Hz, 2H), 2.79 (t, J=6.3Hz, 2H), 1.66-1.86 (m, 4H), 1.22-1.46 (m, 28H), 0.85-0.90 (m, 6H).

IR (KBr)

3252, 2920, 2853, 1701, 1651, 1605, 1435, 1292, 1254, 1111 $cm^{-1}$

Melting point: 106.2-107.2°C

Example 7

Preparation of 4-(6-(4-chlorophenoxy)hexyloxyl)-N-(7-decyloxy-4-chromanon-8-yl)benzamide (Compound 107)

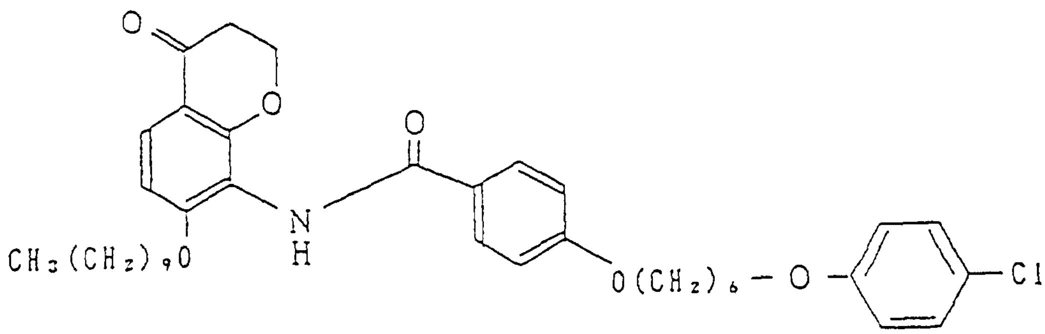

The same procedure was performed as in Example 1, except that use was made of 76 mg of 8-amino-7-decyloxy-4-chromanone, to obtain 73 mg of the desired above-identified compound.

$^1$H-NMR ($CDCl_3$) δ/ppm

7.88 (d, J=8.9Hz, 2H), 7.23 (s, 1H), 7.22 (d, J=8.9Hz, 2H), 6.92 (d, J=8.9Hz, 2H), 6.90 (d, J=8.3Hz, 1H), 6.81 (d, J=8.9Hz, 2H), 6.49 (d, J=8.3Hz, 1H), 4.17 (t, J=4.9Hz, 2H), 4.01 (t, J=6.6Hz, 2H), 3.93 (t, J=6.6Hz, 2H), 3.79 (s, 3H), 2.75 (t, J=6.3Hz, 2H), 1.96 (tt, J=6.3Hz, 4.9Hz, 2H), 1.78-1.86 (m, 4H), 1.51-1.57 (m, 4H)

Example 8

Preparation of N-(7-decyloxyl-4-oxo-4H-1-benzopyran8-yl)-2,2-dimethylpropanamide (Compound 108)

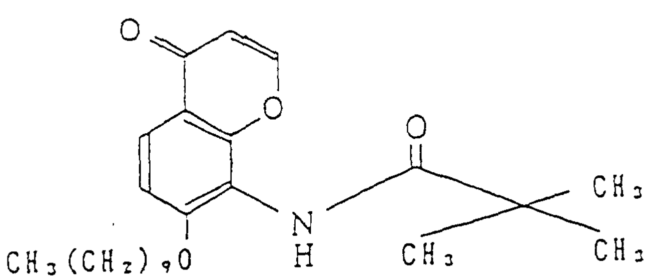

The same procedure was performed as in Example 1, except that use was made of 301 mg of 8-amino-7-decyloxychromon, to obtain 200 mg of the desired above-identified compound.

$^1$H-NMR ($CDCl_3$) δ/ppm

8.10 (d, J=8.9Hz, 1H), 7.79 (d, J=6.3 Hz, 1H), 7.01 (d, J=8.9Hz, 1H), 6.99 (br, 1H), 6.26 (d, J=6.3Hz, 1H), 4.10 (t, J=6.3Hz, 2H), 1.82 (tt, J=6.3, 6.3Hz, 2H), 1.38 (s, 9H), 1.15-1.56 (m, 14H), 0.89 (t, J=6.6Hz, 3H).

IR (KBr)

3281, 2924, 2855, 1664, 1528, 1441, 1406, 1304, 1254, 1086, 804 $cm^{-1}$

Melting point: 108.5-109.0°C

Example 9

Preparation of N-(7-(4-(4-methoxyphenyl)butoxy)-4-chromanon-8-yl)-2,2-dimethylpropanamide (Compound 109)

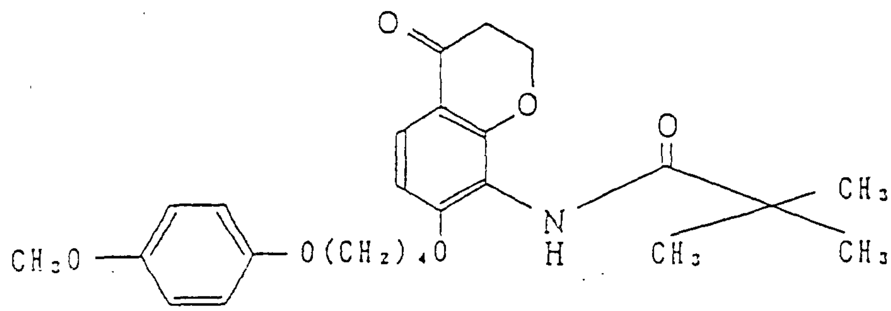

The same procedure was performed as in Example 1, except that use was made of 45 mg of 8-amino-7-(4-(4-methoxyphenyl)butoxy)chromanone, to obtain 34 mg of the desired above-identified compound.

$^1$H-NMR ($CDCl_3$) δ/ppm

7.83 (d, J=8.9Hz, 1H), 6.82 (s, 4H), 6.77 (br.s, 1H), 6.63 (d, J=8.9Hz, 1H), 4.54 (t, J=6.3Hz, 2H), 4.12 (t, J=5.6Hz, 2H), 3.96 (t, J=5.6Hz, 2H), 3.77 (s, 3H), 2.78 (t, J=6.3Hz, 2H), 1.86-2.06 (m, 4H), 1.33 (s, 9H)

Melting point: 119-121°C

Example 10

Preparation of N-(7-(6-phenylhexyloxy)-4-chromanon-8-yl)-2,2-dimethylpropanamide (Compound 110)

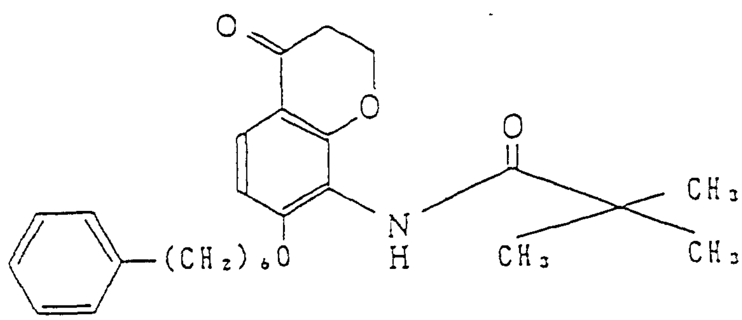

The same procedure was performed as in Example 1, except that use was made of 163 mg of 8-amino-7-(6-phenylhexyloxy)chromanone, to obtain 195 mg of the desired above-identified compound.

$^1$H-NMR ($CDCl_3$) δ/ppm

7.82 (d, J=8.9Hz, 1H), 7.15-7.30 (m, 5H), 6.60 (d, J=8.9Hz, 1H), 4.53 (t, J=6.3Hz, 2H), 4.02 (t, J=6.3Hz, 2H), 2.77 (t, J=6.3Hz, 2H), 2.61 (t, J=7.6Hz, 2H), 1.77 (tt, J=6.6 and 6.3Hz, 2H), 1.64 (tt, J=7.6 and 7.3Hz, 2H), 1.22-1.57 (m, 4H), 1.33 (s, 9H,

Melting point: 140-141°C

Example 11

Preparation of N-(7-methoxy-4-chromanon-8-yl)-4-(6-(4-chlorophenoxy)hexyloxy)benzamide (Compound 201)

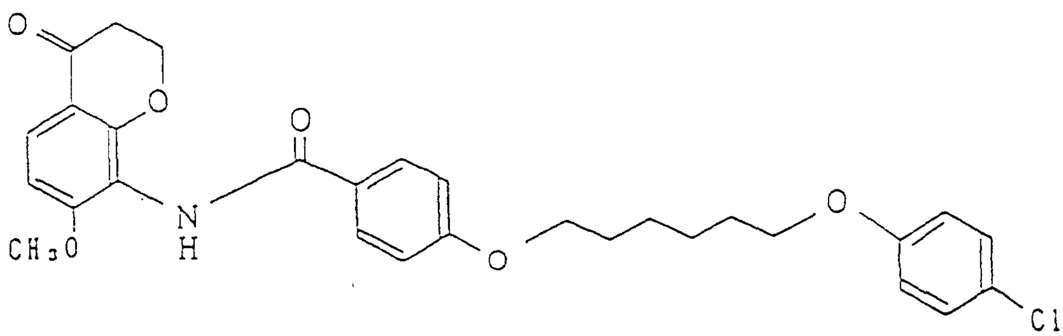

A 100 mL amount of thionyl chloride was added to 9.10 g of 6-(4-chlorophenoxy)hexyloxybenzoic acid and the mixture was stirred for 2 hours under reflux. The excess thionyl chloride was distilled off under reduced pressure and the residue was dissolved in 100 mL of 1,1,2-trichloroethane.

A solution of 5.04 g of 7-methoxy-8-amino-4-chromanone and 2.64 g of triethylamine in 50 mL of-1,1,2-trichloroethane was gradually added in small portions and the mixture was stirred for 72 hours at room temperature.

After the reaction, extraction was performed using dichloromethane. Drying was performed, then the organic solvent was distilled off. The precipitated solid was recrystallized from 750 mL of ethyl acetate, thereby obtaining 9.67 g of the above-identified compound.

$^1$H-NMR ($CDCl_3$) δ/ppm

7.89 (d, J=8.9Hz, 2H), 7.88 (d, J=8.9Hz, 1H), 7.22 (d, J=8.9Hz, 2H), 7.21 (s, 1H), 6.96 (d, J=8.9Hz, 2H), 6.82 (d, J=8.9Hz, 2H), 6.69 (d, J=8.9Hz, 2H), 4.55 (t, J=6.6Hz, 2H), 4.04 (t, J=6.6Hz, 2H), 3.95 (t, J=6.6Hz, 2H), 3.90 (s, 3H), 2.79 (t, J=6.6Hz, 2H), 1.79-1.85 (m, 4H), 1.54-1.57 (m, 4H)

Melting point: 163-164°C

Example 12

Preparation of N-(7-methoxy-4-chromanon-8-yl)-4-(4-chlorobenzyloxy)benzamide (Compound 202)

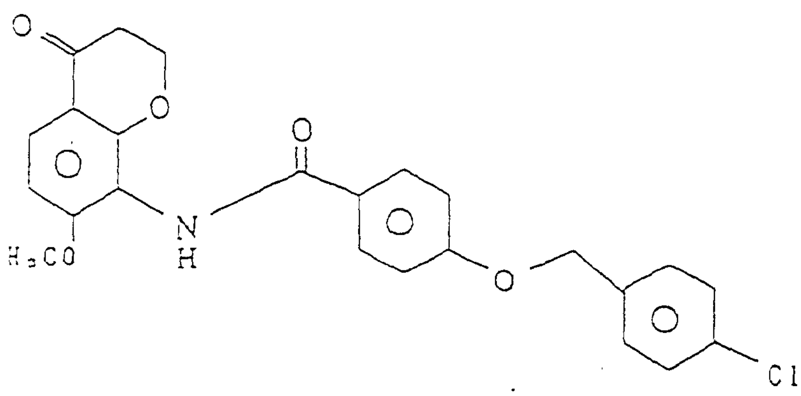

A 53 mg amount of N-(7-methoxy-4-chromanon-8-yl)-4-hydroxybenzamide was dissolved in 2 mL of acetonitrile and 35 mg of potassium carbonate was added.

A 50 ul amount of 4-chlorobenzyl chloride was added while stirring the mixture at room temperature and a reaction was effected for 6 hours.

After the reaction, the potassium carbonate was removed and purification was performed by column chromatography to obtain 64 mg (87%) of the above-identified compound.

$^1$H-NMR ($CDCl_3$) δ/ppm

7.90 (d, J=8.7Hz), 7.89 (d, 1H, J=9.1Hz), 7.38 (s, 4H), 7.24 (brs. 1H), 7.03 (d, 2H, J=8.7Hz), 6.69 (d, 1H, J=9.lHz), 5.11 (s, 2H), 4.55 (t, 2H, J=6.4Hz), 3.90 (s, 3H), 2.79 (t, 2H, J=6.4Hz).

Melting point: 91-92°C

Example 13

Preparation of N-(7-methoxy-4-chromanon-8-yl)-4-(6-(2-methylpropyloxy)hexyloxy)benzamide (Compound 203)

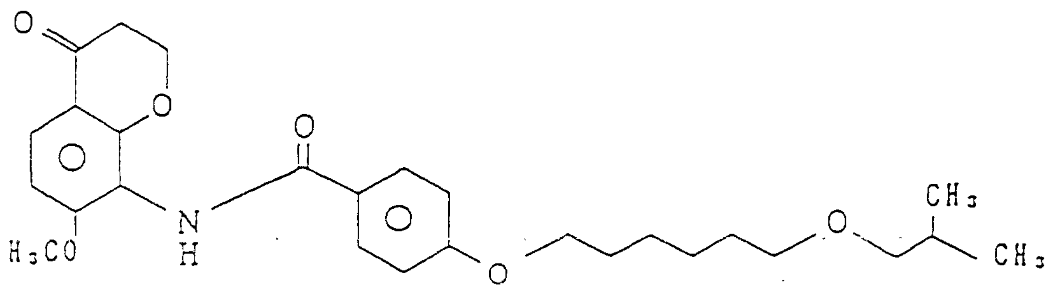

6-(2-Methylpropoxy)hexyl bromide was used and caused to react with 60 mg of N-(7-methoxy-4-chromanon-8-yl)-4-hydroxybenzamide in the same way as in Example 12 to obtain 69.6 mg of the desired above-identified compound.

$^1$H-NMR ($CDCl_3$) δ/ppm

0.90 (d, 6H, J=7Hz), 1.4-1.9 (m, 9H), 2.79 (t, 2H, J=7Hz), 3.17 (d, 2H, J=7Hz), 3.42 (t, 2H, J=7Hz), 3.90 (s, 3H), 4.03 (t, 2H, J=7Hz), 4.55 (t, 2H, J=7Hz), 6.69 (d, 1H, J=8Hz), 6.95 (d, 2H, J=8Hz), 7.2 (bs, 1H), 7.88 (d, 3H, J=8Hz).

Melting point: 148-150°C

Example 14

Preparation of N-(7-methoxy-4-chromanon-8-yl)-4-(6-propoxyhexyloxy)benzamide (Compound 204)

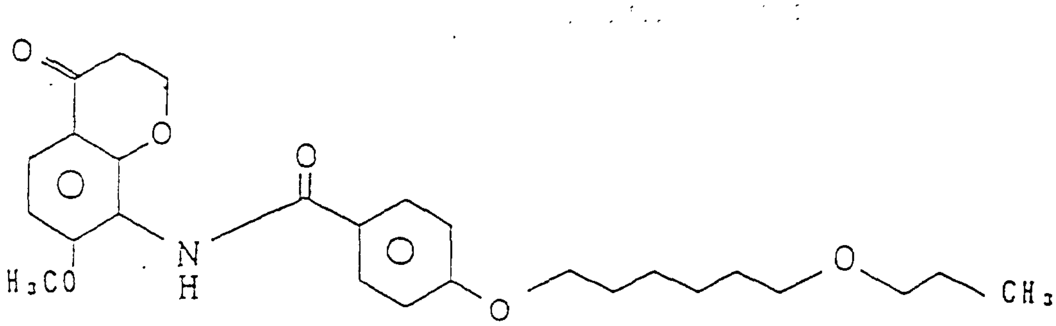

6-Propoxyhexyl bromide was used and allowed to react with 64 mg of N-(7-methoxy-4-chromanon-8-yl)-4-hydroxybenzamide in the same way as in Example 12 to obtain 6.4 mg of the desired above-identified compound.

$^1$H-NMR ($CDCl_3$) δ/ppm

0.92 (t, 3H, J=7Hz), 1.3-1.9 (m, 10H), 2.79 (t, 2H, J=7Hz), 3.37 (t, 2H, J=7Hz), 3.43 (t, 2H, J=7Hz), 3.90 (s, 3H), 4.04 (t, 2H, J=7Hz), 4.55 (t, 2H, J=7Hz), 6.69 (d, 1H, J=9Hz), 6.95 (d, 2H, J=9Hz), 7.21 (bs, 1H), 7.88 (d, 3H, J=9Hz).

Example 15

Preparation of N-(7-methoxy-4-chromanon-8-yl)-4-(3-dimethylamino)propoxybenzamide (Compound 205)

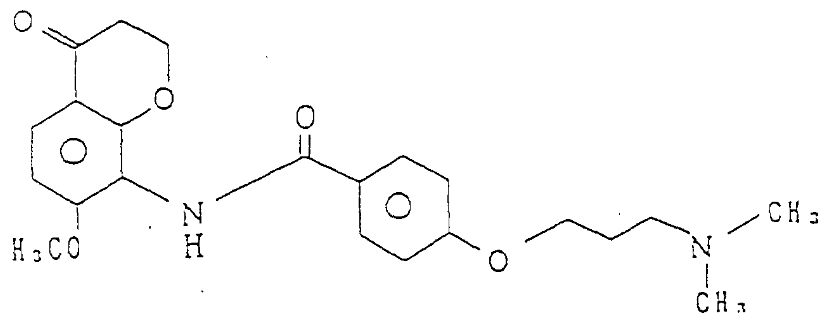

3-Chloro-N,N-dimethylpropylamine was used and allowed to react with 74 mg of N-(7-methoxy-4-chromanon-8-yl)-4-hydroxybenzamide in the same way as in Example 12 to obtain 24 mg of the desired above-identified compound.

$^1$H-NMR ($CDCl_3$, TMS) δ/ppm

7.87-7.89 (m, 3H), 7.21 (brs, 1H), 6.97 (d, 2H, J=9.lHz), 6.69 (d, 1H, J=8.7Hz), 4.55 (t, 2H, J=6.4Hz), 4.09 (t, 2H, J=6.4Hz), 3.90 (s, 3H), 2.79 (t, 2H, J=6.4Hz), 2.46 (t, 2H, J=7.2Hz), 2.26 (s, 6H), 1.99 (tt, 2H, J=6.4Hz, 7.2Hz).

Melting point: 148-149°C

Example 16

Preparation of N-(7-methoxy-4-chromanone-8-yl)-4-(2-piperidion)ethyloxybezamide (Compound 206)

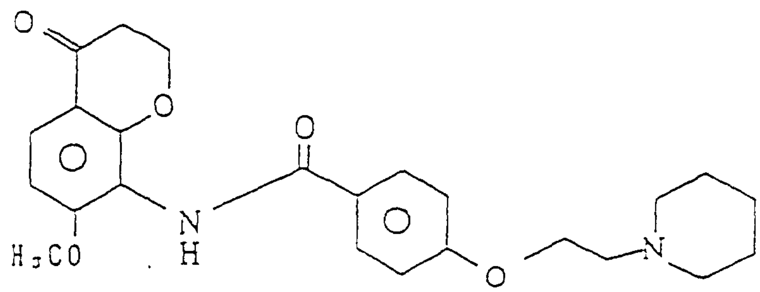

N-(2-chloroethyl)piperidine was used and allowed to react with 50 mg of N-(7-methoxy-4-chromanon-8-yl)-4-hydroxybenzamide in the same way as in Example 12 to obtain 24 mg of the desired above-identified compound.

$^1$H-NMR ($CDCl_3$, TMS) δ/ppm

7.87-7.90 (m, 3H), 7.21 (brs, 1H), 6.98 (d, 2H, J=9.1Hz), 6.69 (d, 1H, J=9.1Hz), 4.55 (t, 2H, J=6.4Hz), 4.18 (t, 2H, J=6.1Hz), 3.90 (s, 3H), 2.82 (t, 2H, J=6.1Hz), 2.79 (t, 2H, J=6.4Hz), 2.54 (br, 4H), 1.60-1.63 (m, 4H), 1.46 (br, 2H)

Reference Example 1

Preparation of 7-decyloxy-4-chromanon-8-yl isocyanate

A 0.14 mL amount of triethylamine was added to a solution of 318 mg of 8-amino-7-decyloxy-4-chromanone and 162 mg of triphosgene in 8 mL of carbon tetrachloride and the mixture was stirred for 3 hours under reflux. The mixture was cooled to room temperature, then ether was added and the insoluble was filtered out and removed. The filtrate was condensed under reduced pressure to obtain 369 mg of the desired above-identified compound.

Example 17

Preparation of 1-(tert-butyl)-3-(7-decyloxy-4-chromanone-8-yl)urea (Compound 126)

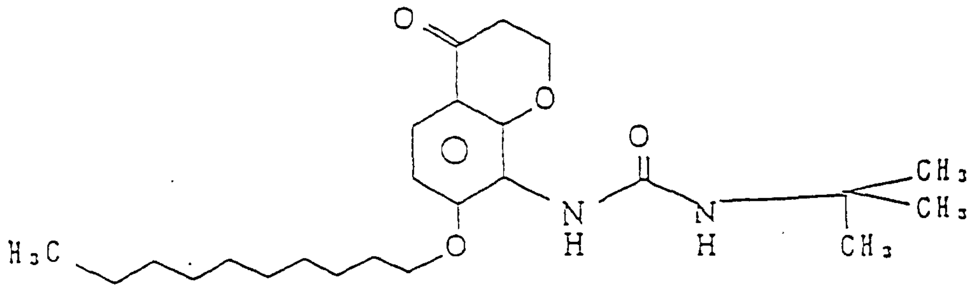

A 30 mg amount of t-butylamine was added to a solution of 106 mg of 7-decyloxy-4-chromanon-8-yl isocyanate in ethyl acetate. The mixture was stirred at room temperature, then the solvent was distilled off and purification was performed by column chromatography (silica gel, hexane/ethyl acetate 1:2), to obtain 119 mg of the desired above-identified compound.

$^1$H-NMR ($CDCl_3$) δ/ppm

7.79 (d, J=8.9Hz, 1H), 6.65 (d, J=8.9Hz, 1H), 5.58 (br.s, 1H), 4.73 (br.s, 1H), 4.57 (t, J=6.3Hz, 2H), 4.07 (t, J=6.6Hz, 2H), 2.78 (t, J=6.3Hz, 2H), 1.82 (tt, J=6.9 and 6.6Hz, 2H), 1.35 (s, 9H), 1.20-1.52 (m, 14H), 0.88 (t, J=6.9Hz, 3H)

Example 18

Measurement of ACAT enzyme inhibitory activity (measurement of ACAT enzyme inhibitory activity of rabbit small intestinal microsomes

Rabbit small intestinal mucosa microsome was prepared and the ACAT enzyme activity was measured by the method of Salone and Field (reference: Biochemica et Biophysica, vol. 712, p. 557 (1982)) with a slight modification.

Using a 40 mM phosphate buffer containing 30 mM EDTA, 50 mM KCl, and 1.0M sucrose and having a pH of 7.4 (buffer A), rabbit small intestinal mucosa was homogenized. This was centrifuged at 10,000 x g and 4°C for 30 minutes to provide a supernatant. The supernatant was further centrifuged at 105,000 x g and 4°C for 1 hour to provide a precipitate. The precipitate was resuspended in the buffer A and used as the microsome fraction.

To the buffer A containing 43 μM bovine serum albumin and 0.5 mg/ml microsome fraction was added various concentrations of each specimen compound (compounds obtained in Examples 1, 3 to 5, and 7 to 14) in dimethylsulfoxide in amounts of 1% v/v. The mixture was heated at 37°C for 5 minutes.

Next, 43μM oleoyl CoA containing [1-$^{14}$C]oleoyl CoA (3.7kBq) was added and the mixture was heated 37°C for 10 minutes, then chloroform/methanol (2/1) containing 10 mg/ml cholesteryl oleate was added to terminate the reaction.

0.111 kBq of [$^3$H] cholesteryl oleate and IN hydrochloric acid were added, and the mixture was stirred. The cholesteryl oleate extracted into the chloroform layer was isolated by a thin layer chromatography, and the radioactivity was measured as the ACAT activity. The results are shown in Table 1.

Table 1:

| ACAT Enzyme Inhibitory Activity | |
|---|---|
| Tested compound | ACAT inhibitory activity $IC_{50}$ (M) |
| Compound described in Ex. 1 | $1.64 \times 10^{-8}$ |
| Compound described in Ex. 3 | $2.70 \times 10^{-8}$ |
| Compound described in Ex. 4 | $8.50 \times 10^{-7}$ |
| Compound described in Ex. 5 | $1.40 \times 10^{-7}$ |
| Compound described in Ex. 7 | $5.40 \times 10^{-7}$ |
| Compound described in Ex. 8 | $1.30 \times 10^{-7}$ |

Table 1: (continued)

| ACAT Enzyme Inhibitory Activity | |
|---|---|
| Tested compound | ACAT inhibitory activity $IC_{50}$ (M) |
| Compound described in Ex. 9 | $4.30 \times 10^{-8}$ |
| Compound described in Ex. 10 | $2.40 \times 10^{-8}$ |
| Compound described in Ex. 11 | $2.60 \times 10^{-8}$ |
| Compound described in Ex. 12 | $2.46 \times 10^{-7}$ |
| Compound described in Ex. 13 | $4.04 \times 10^{-8}$ |
| Compound described in Ex. 14 | $2.46 \times 10^{-7}$ |
| Note that the $LD_{50}$ of the tested compounds was not less than 2 g/kg (mice). | |

Example 19

Measurement of percentage change of serum cholesterol

200 g body weight male Wistar rats were allowed to freely ingest normal feed (CD-2, CLEA Japan Inc.) for 7 days as a preparatory feeding period.

After that, they were allowed to freely ingest feed enriched with cholesterol and fat (2% cholesterol, 1% cholic acid, 20% casein, 45% granulated sugar, 12% coconut oil, 4% KC flock, 1% mixed vitamin, 7% mixed mineral, and 8% dried fish powder, made by CLEA Japan Inc.) for 3 days. During the cholesterol leading period, the test compound of the present invention (compounds obtained in Examples 1 and 11) was administered to the test animals once a day for 3 days in orally in a dosage of 0.3 mg per kg body weight. The control animals were administered only the excipient.

Eight hours after the final administration, the test animals were made to fast. After 16 hours of fasting, the test animals were slaughtered. The serum cholesterol level was measured for each animal.

The results are shown in Table 2 as percentage change of the serum cholesterol in comparison with the control.

$$\text{Percentage change of serum cholesterol (\%)} = ((A- B)/B) \times 100$$

(wherein, A: total serum cholesterol in test compound group, B: total serum cholesterol in control group)

Table 2:

| Percentage of Change of Serum Cholesterol | |
|---|---|
| Test compound | Percentage of change of serum cholesterol (%) |
| Compound of Ex. 1 | -45 |
| Compound of Ex. 11 | -56 |

Example 20

Preparation of Tablet

Tablets containing 30 mg of the compound of Example 1 were produced by the following formula:

| | |
|---|---|
| Compound of Ex. 1 | 30 mg |
| Lactose | 87 mg |
| Starch | 30 mg |
| Magnesium stearate | 3 mg |

According to the present invention, as explained above, it is possible to obtain novel benzopyran derivatives and possible to obtain a drug against atherosclerosis and hyperlipidemia.

## Claims

**1.** Benzopyran derivatives and their pharmacologically acceptable acid adducts of the formula (I):

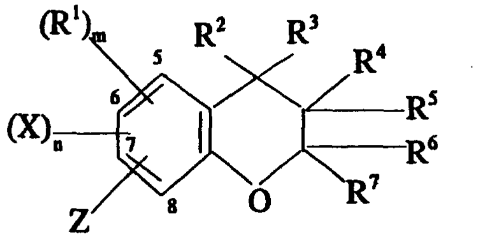

... (I)

wherein

X independently represents an unsubstituted or substituted $C_7$ to $C_{20}$ alkyl group, unsubstituted or substituted $C_7$ to $C_{20}$ alkoxy group, unsubstituted or substituted $C_7$ to $C_{20}$ acylamino group, unsubstituted or substituted $C_{13}$ to $C_{30}$ alkylamino group, unsubstituted or substituted $C_8$ to $C_{20}$ alkyloxycarbonyl group, unsubstituted or substituted $C_8$ to $C_{20}$ acyl group, unsubstituted or substituted $C_{13}$ to $C_{20}$ acyloxy group, unsubstituted or substituted $C_6$ to $C_{20}$ aryl group, or unsubstituted or substituted $C_6$ to $C_{20}$ aryloxy group, in which when X is substituted the substituent group is selected from halogen, hydroxy, amino, alkoxy and aryloxy groups;
Z represents the group:

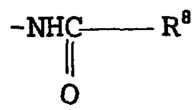

wherein $R^1$ independently represents a hydrogen atom, halogen atom, unsubstituted or substituted $C_1$ to $C_6$ lower alkyl group, unsubstituted or substituted $C_1$ to $C_6$ alkoxy group, hydroxyl group, nitro group, cyano group, unsubstituted or substituted $C_1$ to $C_6$ acylamino group, unsubstituted or substituted $C_1$ to $C_{12}$ alkylamino group, unsubstituted or substituted $C_1$ to $C_7$ lower alkyloxycarbonyl group, carboxyl group, unsubstituted or substituted $C_1$ to $C_7$ acyl group, or unsubstituted or substituted $C_1$ to $C_{12}$ acyloxy group, in which when R' is substituted the substituent group is selected from halogen, hydroxy and amino groups;
$R^2$ and $R^3$ together represent an oxygen atom;
$R^4$ to $R^7$ each represent a hydrogen atom, or
$R^4$ and $R^6$ are combined together to form a carbon-carbon bond;
$R^8$ represents a $C_5$ to $C_{20}$ cycloalkyl or cycloalkenyl group, an unsubstituted or substituted group $C_6$ to $C_{20}$ aryl group, in which the substituent group is selected from halogen, $C_1$ to $C_{14}$ alkyl, $C_1$ to $C_{14}$ alkoxy, acylamino, mono- or di-alkylamino, alkoxycarbonyl, acyl, and acyloxy groups, which may be substituted with substituents selected from halogen, hydroxy, nitro, cyano, $C_1$ to $C_{10}$ alkoxy, $C_6$ to $C_{10}$ aryloxy and $C_1$ to $C_{12}$ alkylamino groups, or $R_8$ represents a group of the formula (VII):

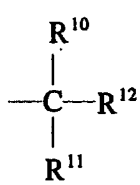

(VII)

wherein, $R^{10}$ and $R^{11}$ independently represent a hydrogen atom, or $C_1$ to $C_6$ alkyl group, $R^{12}$ represents a hydrogen atom, unsubstituted or substituted $C_1$ to $C_{19}$ alkyl or alkenyl group, unsubstituted or substituted to $C_6$ to $C_{19}$ aryl group, unsubstituted or substituted $C_6$ to $C_{19}$ aryl alkyl group, or group of the formula -A-Z-B,

wherein A represents a $C_1$ to $C_{12}$ alkyl group, Z represents an oxygen atom or sulfur atom, and B represents an unsubstituted $C_1$ to $C_{19}$ alkyl group, unsubstituted or substituted $C_6$ to $C_{19}$ aryl group, or unsubstituted or substituted $C_6$ to $C_{19}$ aryl alkyl group in which when $R^{12}$ or B is substituted the substituent group is selected from halogen, $C_1$ to $C_6$ alkyl, $C_1$ to $C_6$ alkoxy, hydroxy, nitro, cyano, $C_1$ to $C_6$ acylamino, $C_1$ to $C_{12}$ alkylamino, $C_1$ to $C_7$ alkoxycarbonyl, carboxyl, $C_1$ to $C_7$ acyl and $C_1$ to $C_{12}$ acyloxycarbonyl groups or $R^8$ represents a group of formula (IX):

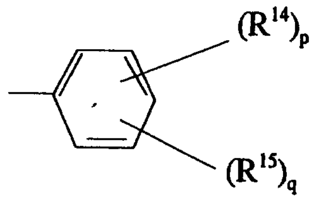

(IX)

wherein $R^{14}$ independently represents a substituted $C_1$ to $C_{14}$ alkyl group, substituted $C_1$ to $C_{14}$ alkoxy group, substituted $C_1$ to $C_{14}$ acylamino group, substituted $C_1$ to $C_{14}$ alkylamino group, substituted $C_1$ to $C_{14}$ alkoxycarbonyl group, substituted $C_1$ to $C_{14}$ acyl group, or substituted $C_1$ to $C_{14}$ acyloxy group in which the substituent is selected from halogen, $C_1$ to $C_{13}$ alkyl, $C_1$ to $C_{13}$ alkoxy, $C_6$ to $C_{13}$ aryloxy, $C_6$ to $C_{13}$ aryl, $C_1$ to $C_{13}$ acylamino, $C_5$ to $C_8$ cyclic amino, $C_1$ to $C_{13}$ mono or dialkylamino, $C_2$ to $C_{13}$ alkyloxycarbonyl, $C_1$ to $C_{13}$ acyl, $C_1$ to $C_{14}$ acyloxy, hydroxy, amino, nitro, cyano and carboxyl groups, in which the $C_1$ to $C_{13}$ alkyl group, $C_1$ to $C_{13}$ alkoxy group, $C_6$ to $C_{13}$ aryloxy group, $C_6$ to $C_{13}$ aryl group and $C_1$ to $C_{13}$ mono or dialkylamino groups may be substituted with substituents selected from halogen, $C_1$ to $C_{13}$ alkyl, $C_1$ to $C_{13}$ alkoxy, $C_6$ to $C_{13}$ aryloxy, $C_6$ to $C_{13}$ aryl, $C_1$ to $C_{13}$ acylamino, $C_5$ to $C_8$ cyclic amino, $C_1$ to $C_{13}$ mono or dialkylamino, $C_2$ to $C_{13}$ alkyloxycarbonyl, $C_1$ to $C_{13}$ acyl, $C_1$ to $C_{14}$ acyloxy, hydroxy, amino, nitro, cyano and carboxyl groups; $R^{15}$ independently represents a hydrogen atom, halogen atom, unsubstituted $C_1$ to $C_{14}$ alkyl group, unsubstituted $C_1$ to $C_{14}$ alkoxy group, unsubstituted $C_1$ to $C_{14}$ acylamino group, unsubstituted $C_1$ to $C_{14}$ alkylamine group, unsubstituted $C_1$ to $C_{14}$ alkyloxycarbonyl group, unsubstituted $C_1$ to $C_{14}$ acyl group, unsubstituted $C_1$ to $C_{14}$ acyloxy group; p is an integer of 1 to 5; q is an integer of 0 to 4; and p and q together do not exceed 5; and m is an integer of 0 to 3, n is 0 or 1, and m and n together do not exceed 3, but when n = 0, $R^8$ represents the group of formula (IX), and when n = 1 the substitution position of X is the 6-position or 7-position of the benzopyran ring.

**2.** Benzopyran derivatives and their pharmacologically acceptable acid adducts according to claim 1, wherein the substitution position of the group Z is the 8-position or 5-position of the benzopyran ring.

**3.** Benzopyran derivatives and their pharmacologically acceptable acid adducts according to claim 2, wherein the substitution position of the group Z is the 8-position of the benzopyran ring.

**4.** Benzopyran derivatives and their pharmacologically acceptable acid adducts according to claim 3, wherein there is a substituent group other than a hydrogen atom at the 7-position of the benzopyran ring.

**5.** Benzopyran derivatives and their pharmacologically acceptable acid adducts according to claim 4, wherein the substituent group of the 7-position of the benzopyran ring is an unsubstituted or substituted $C_7$ to $C_{20}$ alkyl group or unsubstituted or substituted $C_7$ to $C_{20}$ alkoxy group.

**6.** Benzopyran derivatives and their pharmacologically acceptable acid adducts according to claim 5, wherein the substituent group of the 7-position of the benzopyran ring is an unsubstituted or substituted $C_7$ to $C_{20}$ alkoxy group.

**7.** Benzopyran derivatives and their pharmacologically acceptable acid adducts according to claim 1, wherein n in the formula (I) is 0 and $R^8$ is the group of the formula (IX).

**8.** Benzopyran derivatives and their pharmacologically acceptable acid adducts according to claim 7, wherein the substitution position of the group Z is the 8-position of the benzopyran ring.

**9.** Benzopyran derivatives and their pharmacologically acceptable acid adducts according to claim 8, wherein there is a substituent group other than hydrogen atom at the 7-position of the benzopyran ring.

**10.** A pharmaceutical composition comprising an effective amount of a benzopyran derivative or pharmacologically acceptable acid adduct thereof according to any of claims 1 to 9, and a pharmacologically-acceptable carrier.

**11.** An ACAT inhibitor in unit dosage form having as an essential ingredient an ACAT inhibitory effective amount of a benzopyran derivative or pharmacologically acceptable acid adduct thereof according to any of claims 1 to 9, and a pharmacologically-acceptable carrier.

**12.** A drug for arteriosclerosis in unit dosage form having as an essential ingredient an anti-arteriosclerosis effective amount of a benzopyran derivative or a pharmacologically acceptable acid adduct thereof according to any of claims 1 to 9, and a pharmacologically-acceptable carrier.

**13.** A drug for hyperlipidemia in unit dosage form having as an essential ingredient an anti-hyperlipidemia effective amount of a benzopyran derivative or pharmacologically acceptable acid adduct thereof according to any of claims 1 to 9, and a pharmacologically-acceptable carrier.

**Patentansprüche**

**1.** Benzopyran-Derivate und deren pharmakologisch geeignete Säureaddukte der Formel (I):

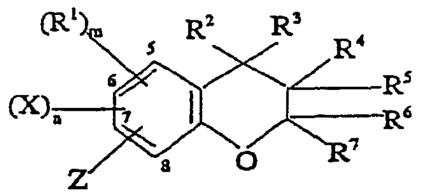

... (I)

worin

X unabhängig eine unsubstituierte oder substituierte $C_7$-$C_{20}$-Alkylgruppe, eine unsubstituierte oder substituierte $C_7$-$C_{20}$-Alkoxygruppe, eine unsubstituierte oder substituierte $C_7$-$C_{20}$-Acylaminogruppe, eine unsubstituierte oder substituierte $C_{13}$-$C_{30}$-Alkylaminogruppe, eine unsubstituierte oder substituierte $C_8$-$C_{20}$-Alkyloxycarbonyl-Gruppe, eine unsubstituierte oder substituierte $C_8$-$C_{20}$-Acylgruppe, eine unsubstituierte oder substituierte $C_{13}$-$C_{20}$-Acyloxygruppe, eine unsubstituierte oder substituierte $C_6$-$C_{20}$-Arylgruppe oder eine unsubstituierte oder substituierte $C_6$-$C_{20}$-Aryloxygruppe repräsentiert, in welchen, wenn X substituiert ist, die Substituentengruppe ausgewählt ist aus Halogen-, Hydroxy-, Amino-, Alkoxy- und Aryloxygruppen;

worin Z die Gruppe:

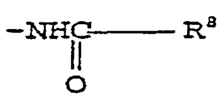

repräsentiert;

worin $R^1$ unabhängig ein Wasserstoffatom, ein Halogenatom, eine unsubstituierte oder substituierte $C_1$-$C_6$-Niederalkylgruppe, eine unsubstituierte oder substituierte $C_1$-$C_6$-Alkoxygruppe, eine Hydroxylgruppe, eine Nitrogruppe, eine Cyanogruppe, eine unsubstituierte oder substituierte $C_1$-$C_6$-Acylaminogruppe, eine unsubstituierte oder substituierte $C_1$-$C_{12}$-Alkylaminogruppe, eine unsubstituierte oder substituierte $C_1$-$C_7$-Niederalkyloxycarbonyl-Gruppe, eine Carboxylgruppe, eine unsubstituierte oder substituierte $C_1$-$C_7$-Acylgruppe, eine unsubstituierte oder substituierte $C_1$-$C_{12}$-Acyloxygruppe repräsentiert, in welchen, wenn $R^1$ substituiert ist, die Substituentengruppe ausgewählt ist aus Halogen-, Hydroxy- und Aminogruppen;

worin $R^2$ und $R^3$ zusammen ein Sauerstoffatom repräsentieren;

worin $R^4$ bis $R^7$ jeweils ein Wasserstoffatom repräsentieren, oder

worin $R^4$ und $R^6$ miteinander kombiniert eine Kohlenstoff-Kohlenstoff-Bindung bilden;

worin $R^8$ eine $C_5$-$C_{20}$-Cykloalkyl- oder -Cykloalkenylgruppe, eine unsubstituierte oder substituierte Gruppe des $C_6$-$C_{20}$-Arylgruppen-Typs, in welchem die Substituentengruppe ausgewählt ist aus Halogen-, $C_1$-$C_{14}$-Alkyl-, $C_1$-$C_{14}$-Alkoxy-, Acylamino-, Mono- oder Dialkylamino-, Alkoxycarbonyl-, Acyl-, und Acyloxygruppen, welche mit Substituenten substituiert sein können, welche ausgewählt sind aus Halogen-, Hydroxy-, Nitro-, Cyano-, $C_1$-$C_{10}$-Alkoxy-, $C_6$-$C_{10}$-Aryloxy- und $C_1$-$C_{12}$-Alkylaminogruppen oder worin $R^8$ eine Gruppe der Formel (VII) repräsentiert:

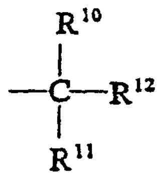

(VII)

worin $R^{10}$ und $R^{11}$ unabhängig voneinander ein Wasserstoffatom oder eine $C_1$-$C_6$-Alkylgruppe repräsentieren, worin $R^{12}$ ein Wasserstoffatom, eine unsubstituierte oder substituierte $C_1$-$C_{19}$-Alkyl- oder Alkenylgruppe, eine unsubstituierte oder substituierte $C_6$-$C_{19}$-Arylgruppe, eine unsubstituierte oder substituierte $C_6$-$C_{19}$-Arylalkylgruppe oder eine Gruppe der Formel -A-Z-B repräsentiert, worin A eine $C_1$-$C_{12}$-Alkylgruppe repräsentiert, worin Z ein Sauerstoffatom oder ein Schwefelatom repräsentiert und B eine unsubstituierte $C_1$-$C_{19}$-Alkylgruppe, eine unsubstituierte oder substituierte $C_6$-$C_{19}$-Arylgruppe oder eine unsubstituierte oder substituierte $C_6$-$C_{19}$-Arylalkylgruppe repräsentiert, in welcher, falls $R^{12}$ oder B substituiert ist, die Substituentengruppe ausgewählt ist aus Halogen-, $C_1$-$C_6$-Alkyl-, $C_1$-$C_6$-Alkoxy-, Hydroxy-, Nitro-, Cyano-, $C_1$-$C_6$-Acylamino-, $C_1$-$C_{12}$-Alkylamino-, $C_1$-$C_7$-Alkoxycarbonyl-, Carboxyl-, $C_1$-$C_7$-Acyl- und $C_1$-$C_{12}$-Acyloxycarbonylgruppen oder $R^8$ repräsentiert eine Gruppe der Formel (IX):

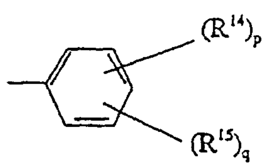

(IX)

worin $R^{14}$ unabhängig eine substituierte $C_1$-$C_{14}$-Alkylgruppe, eine substituierte $C_1$-$C_{14}$-Alkoxygruppe, eine $C_1$-$C_{14}$-Acylaminogruppe, eine substituierte $C_1$-$C_{14}$-Alkylaminogruppe, eine substituierte $C_1$-$C_{14}$-Alkoxycarbonylgruppe, eine substituierte $C_1$-$C_{14}$-Acylgruppe oder eine substituierte $C_1$-$C_{14}$-Acyloxygruppe repräsentiert, in welchen der Substituent ausgewählt ist aus Halogen-, $C_1$-$C_{13}$-Alkyl-, $C_1$-$C_{13}$-Alkoxy-, $C_6$-$C_{13}$-Aryloxy-, $C_6$-$C_{13}$-Aryl-, $C_1$-$C_{13}$-Acylamino-, zyklische $C_5$-$C_8$-Amino-, $C_1$-$C_{13}$-Mono-oder Dialkylamino-, $C_2$-$C_{13}$-Alkyloxycarbonyl-, $C_1$-$C_{13}$-Acyl-, $C_1$-$C_{14}$-Acyloxy-, Hydroxy-, Amino-, Nitro-, Cyano- und Carboxylgruppen, in welchen die $C_1$-$C_{13}$-Alkylgruppe, die $C_1$-$C_{13}$-Alkoxygruppe, die $C_6$-$C_{13}$-Aryloxygruppe, die $C_6$-$C_{13}$-Arylgruppe und die $C_1$-$C_{13}$-Mono- oder Dialkylaminogruppen substituiert sein können mit Substituenten, welche ausgewählt sind aus Halogen-, $C_1$-$C_{13}$-Alkyl-, $C_1$-$C_{13}$-Alkoxy-, $C_6$-$C_{13}$-Aryloxy-, $C_6$-$C_{13}$-Aryl-, $C_1$-$C_{13}$-Acylamino-, zyklischen $C_5$-$C_8$-Amino-, $C_1$-$C_{13}$-Mono- oder -Dialkylamino-, $C_2$-$C_{13}$-Alkyloxycarbonyl-, $C_1$-$C_{13}$-Acyl-, $C_1$-$C_{14}$-Acyloxy-, Hydroxy-, Amino-, Nitro-, Cyano- und Carboxylgruppen; worin $R^{15}$ unabhängig ein Wasserstoffatom, ein Halogenatom, eine unsubstituierte $C_1$-$C_{14}$-Alkylgruppe, eine unsubstituierte $C_1$-$C_{14}$-Alkoxygruppe, eine unsubstituierte $C_1$-$C_{14}$-Acylaminogruppe, eine unsubstituierte $C_1$-$C_{14}$-Alkylaminogruppe, eine unsubstituierte $C_1$-$C_{14}$-Alkyloxycarbonylgruppe, eine unsubstituierte $C_1$-$C_{14}$-Acylgruppe, eine unsubstituierte $C_1$-$C_{14}$-Acyloxygruppe repräsentiert; worin p eine ganze Zahl von von 1 bis 5 ist; worin q eine ganze Zahl von 0 bis 4 ist; und worin p und q zusammen 5 nicht überschreiten; und worin m eine ganze Zahl von 0 bis 3 ist, n gleich 0 oder 1 ist und m und n zusammen 3 nicht überschreiten, wenn jedoch n = 0 ist, repräsentiert $R^8$ eine Gruppe der Formel (IX), und wenn n = 1 ist, ist die Substitutionsposition von X die 6-Position oder 7-Position des Benzopyran-Ringes.

**2.** Benzopyran-Derivate und deren pharmakologisch geeignete Säureaddukte nach Anspruch 1, worin die Substitutionsposition der Z-Gruppe die 8-Position oder die 5-Position des Benzopyran-Ringes ist.

**3.** Benzopyran-Derivate und deren pharmakologisch geeignete Säureaddukte nach Anspruch 2, worin die Substitutionsposition der Z-Gruppe die 8-Position des Benzopyran-Ringes ist.

**4.** Benzopyran-Derivate und deren pharmakologisch geeignete Säureaddukte nach Anspruch 3, worin eine Substituentengruppe, die von einem Wasserstoffatom verschieden ist, an der 7-Position des Benzopyran-Ringes vorhanden ist.

**5.** Benzopyran-Derivate und deren pharmakologisch geeignete Säureaddukte nach Anspruch 4, worin die Substituentengruppe der 7-Position des Benzopyran-Ringes eine unsubstituierte oder substituierte $C_7$-$C_{20}$-Alkylgruppe oder eine unsubstituierte oder substituierte $C_7$-$C_{20}$-Alkoxygruppe ist.

**6.** Benzopyran-Derivate und deren pharmakologisch geeignete Säureaddukte nach Anspruch 5, worin die Substituentengruppe der 7-Position des Benzopyran-Ringes eine unsubstituierte oder substituierte $C_7$-$C_{20}$-Alkoxygruppe ist.

**7.** Benzopyran-Derivate und deren pharmakologisch geeignete Säureaddukte nach Anspruch 1, worin n in der Formel (I) 0 ist und R8 die Gruppe der Formel (IX) ist.

**8.** Benzopyran-Derivate und deren pharmakologisch geeignete Säureaddukte nach Anspruch 7, worin die Substitutionsposition der Z-Gruppe die 8-Position des Benzopyran-Ringes ist.

**9.** Benzopyran-Derivate und deren pharmakologisch geeignete Säureaddukte nach Anspruch 8, worin an der 7-Position des Benzopyran-Ringes eine Substituentengruppe vorhanden ist, welche von einem Wasserstoffatom verschieden ist.

**10.** Pharmazeutische Zusammensetzung, umfassend eine wirksame Menge eines Benzopyran-Derivates oder eines pharmakologisch geeigneten Säureadduktes hiervon nach einem der Ansprüche 1 bis 9 und einen pharmakologisch geeigneten Träger.

**11.** ACAT-Inhibitor in Form einer Dosierungseinheit, welche als essenziellen Inhaltsstoff eine Menge eines Benzopyran-Derivates oder eines pharmakologisch geeigneten Säureadduktes hiervon gemäß einem der Ansprüche 1 bis 9 in einer ACAT-inhibierend wirksamen Menge und einen pharmakologisch geeigneten Trägerstoff enthält.

**12.** Arzneimittel für Arteriosklerose in Form einer Dosierungseinheit mit einem essenziellen Inhaltsstoff in einer antiarteriosklerosis-wirksamen Menge eines Benzopyran-Derivates oder eines pharmakologisch geeigneten Säureadduktes hiervon gemäß einem der Ansprüche 1 bis 9 sowie einem pharmakologisch geeigneten Trägerstoff.

**13.** Arzneimittel für Hyperlipidämie in Form einer Dosierungseinheit mit einem essenziellen Inhaltsstoff eines Benzopyran-Derivates oder eines pharmakologisch geeigneten Säureadduktes hiervon gemäß einem der Ansprüche 1 bis 9 in antihyperlipidämie-wirksamer Menge und einem pharmakologisch geeigneten Trägerstoff.

**Revendications**

**1.** Dérivés de benzopyrane et leurs produits d'addition d'acide acceptables d'un point de vue pharmacologique, de formule (I)

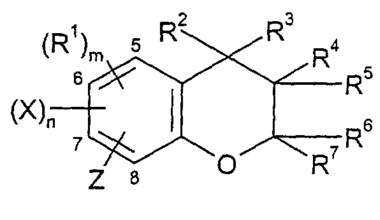

... (I)

dans laquelle

X représente indépendamment un groupe alkyle en $C_7$ à $C_{20}$ substitué ou non substitué, un groupe alcoxy en $C_7$ à $C_{20}$ substitué ou non substitué, un groupe acylamino en $C_7$ à $C_{20}$ substitué ou non substitué, un groupe alkylamino en $C_{13}$ à $C_{30}$ substitué ou non substitué, un groupe alkyloxycarbonyle en $C_8$ à $C_{20}$ substitué ou non substitué, un groupe acyle en $C_8$ à $C_{20}$ substitué ou non substitué, un groupe acyloxy en $C_{13}$ à $C_{20}$ substitué ou non substitué, un groupe aryle en $C_6$ à $C_{20}$ substitué ou non substitué, ou un groupe aryloxy en $C_6$ à $C_{20}$ substitué ou non substitué, dans lequel, lorsque X est substitué, le groupe substituant est choisi parmi les halogènes et les radicaux hydroxy, amino, alcoxy et aryloxy ;
Z représente le groupe :

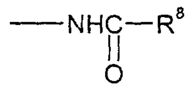

dans laquelle $R^1$ représente indépendamment un atome d'hydrogène, un atome d'halogène, un groupe alkyle inférieur en $C_1$ à $C_6$ substitué ou non substitué, un groupe alcoxy en $C_1$ à $C_6$ substitué ou non substitué, un groupe hydroxyle, un groupe nitro, un groupe cyano, un groupe acylamino en $C_1$ à $C_6$ substitué ou non substitué, un groupe alkylamino en $C_1$ à $C_{12}$ substitué ou non substitué, un groupe alkyloxycarbonyle inférieur en $C_1$ à $C_7$ substitué ou non substitué, un groupe carboxyle, un groupe acyle en $C_1$ à $C_7$ substitué ou non substitué, ou un groupe acyloxy en $C_1$ à $C_{12}$ substitué ou non substitué, dans lequel, lorsque $R^1$ est substitué, le groupe substituant est choisi parmi les halogènes et les groupes hydroxy et amino ;
$R^2$ et $R^3$ représentent ensemble un atome d'oxygène ;
$R^4$ à $R^7$ représentent chacun un atome d'hydrogène, ou
$R^4$ et $R^6$ sont combinés ensemble pour former une liaison carbone-carbone ;
$R^8$ représente un groupe cycloalkyle ou cycloalcényle en $C_5$ à $C_{20}$, un groupe aryle en $C_6$ à $C_{20}$ substitué ou non substitué dans lequel le groupe substituant est choisi parmi les halogènes, les groupes alkyle en $C_1$ à $C_{14}$, alcoxy en $C_1$ à $C_{14}$, acylamino, mono- ou di-alkylamino, alcoxycarbonyle, acyle, et acyloxy, lesquels peuvent être substitués par des substituants choisis parmi les halogènes et les groupes hydroxy, nitro, cyano, alcoxy en $C_1$ à $C_{10}$, aryloxy en $C_6$ à $C_{10}$ et alkylamino en $C_1$ à $C_{12}$, ou bien $R^8$ représente un groupe de formule (VII)

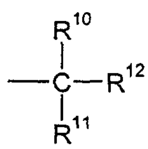

... (VII)

dans laquelle $R^{10}$ et $R^{11}$ représentent indépendamment un atome d'hydrogène ou un groupe alkyle en $C_1$ à $C_6$, $R^{12}$ représente un atome d'hydrogène, un groupe alkyle ou alcényle en $C_1$ à $C_{19}$ substitué ou non substitué, un groupe aryle en $C_6$ à $C_{19}$ substitué ou non substitué, un groupe arylalkyle en $C_6$ à $C_{19}$ substitué ou non substitué, ou un groupe de formule -A-Z-B dans laquelle A représente un groupe alkyle en $C_1$ à $C_{12}$, Z représente un atome d'oxygène ou un atome de soufre, et B représente un groupe alkyle en $C_1$ à $C_{19}$ non substitué, un groupe aryle en $C_6$ à $C_{19}$ substitué ou non substitué, ou un groupe arylalkyle en $C_6$ à $C_{19}$ substitué ou non substitué, dans lequel, quand $R^{12}$ ou B est substitué, le groupe substituant est choisi parmi les halogènes et les groupes alkyle en $C_1$ à $C_6$, alcoxy en $C_1$ à $C_6$, hydroxy, nitro, cyano, acylamino en $C_1$ à $C_6$, alkylamino en $C_1$ à $C_{12}$, alcoxycarbonyle en $C_1$ à $C_7$, carboxyle, acyle en $C_1$ à $C_7$ et acyloxycarbonyle en $C_1$ à $C_{12}$, ou bien $R^8$ représente un groupe de formule (IX) :

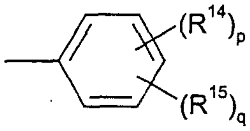

... (IX)

dans laquelle $R^{14}$ représente indépendamment un groupe alkyle en $C_1$ à $C_{14}$ substitué, un groupe alcoxy en

$C_1$ à $C_{14}$ substitué, un groupe acylamino en $C_1$ à $C_{14}$ substitué, un groupe alkylamino en $C_1$ à $C_{14}$ substitué, un groupe alcoxycarbonyle en $C_1$ à $C_{14}$ substitué, un groupe acyle en $C_1$ à $C_{14}$ substitué, ou un groupe acyloxy en $C_1$ à $C_{14}$ substitué, dans lequel le substituant est choisi parmi les halogènes et les groupes alkyle en $C_1$ à $C_{13}$, alcoxy en $C_1$ à $C_{13}$, aryloxy en $C_6$ à $C_{13}$, aryle en $C_6$ à $C_{13}$, acylamino en $C_1$ à $C_{13}$, amino cycliques en $C_5$ à $C_8$, mono- ou di-alkylamino en $C_1$ à $C_{13}$, alkyloxycarbonyle en $C_2$ à $C_{13}$, acyle en $C_1$ à $C_{13}$, acyloxy en $C_1$ à $C_{14}$, hydrox-y, amino, nitro, cyano et carboxyle, et dans lequel le groupe alkyle en $C_1$ à $C_{13}$, le groupe alcoxy en $C_1$ à $C_{13}$, le groupe aryloxy en $C_6$ à $C_{13}$, le groupe aryle en $C_6$ à $C_{13}$ et les groupes mono- ou di-alkylamino en $C_1$ à $C_{13}$ peuvent être substitués par des substituants choisis parmi les halogènes et les groupes alkyle en $C_1$ à $C_{13}$, alcoxy en $C_1$ à $C_{13}$, aryloxy en $C_6$ à $C_{13}$, aryle en $C_6$ à $C_{13}$, acylamino en $C_1$ à $C_{13}$, amino cyclique en $C_5$ à $C_8$, mono- ou di-alkylamino en $C_1$ à $C_{13}$, alkyloxycarbonyle en $C_2$ à $C_{13}$, acyle en $C_1$ à $C_{13}$, acyloxy en $C_1$ à $C_{14}$, hydroxy, amino, nitro, cyano et carboxyle ; $R^{15}$ représente indépendamment un atome d'hydrogène, un atome d'halogène, un groupe alkyle en $C_1$ à $C_{14}$ non substitué, un groupe alcoxy en $C_1$ à $C_{14}$ non substitué, un groupe acylamino en $C_1$ à $C_{14}$ non substitué, un groupe alkylamine en $C_1$ à $C_{14}$ non substitué, un groupe alkyloxycarbonyle en $C_1$ à $C_{14}$ non substitué, un groupe acyle en $C_1$ à $C_{14}$ non substitué, un groupe acyloxy en $C_1$ à $C_{14}$ non substitué ; p est un entier de 1 à 5 ; q est un entier de 0 à 4 ; et p et q ensemble ne dépassent pas 5 ; et m est un entier de 0 à 3, n vaut 0 ou 1, et m et n ensemble ne dépassent pas 3, mais quand n = 0, $R^8$ représente le groupe de formule (IX), et quand n = 1, la position de substitution de X est la position 6 ou la position 7 du cycle benzopyrane.

**2.** Dérivés de benzopyrane et leurs produits d'addition d'acide acceptables d'un point de vue pharmacologique selon la revendication 1, dans lesquels la position de substitution du groupe Z est la position 8 ou la position 5 du cycle benzopyrane.

**3.** Dérivés de benzopyrane et leurs produits d'addition d'acide acceptables d'un point de vue pharmacologique selon la revendication 2, dans lesquels la position de substitution du groupe Z est la position 8 du cycle benzopyrane.

**4.** Dérivés de benzopyrane et leurs produits d'addition d'acide acceptables d'un point de vue pharmacologique selon la revendication 3, dans lesquels il y a un groupe substituant autre qu'un atome d'hydrogène au niveau de la position 7 du cycle benzopyrane.

**5.** Dérivés de benzopyrane et leurs produits d'addition d'acide acceptables d'un point de vue pharmacologique selon la revendication 4, dans lesquels le groupe substituant au niveau de la position 7 du cycle benzopyrane est un groupe alkyle en $C_7$ à $C_{20}$ substitué ou non substitué ou un groupe alcoxy en $C_7$ à $C_{20}$ substitué ou non substitué.

**6.** Dérivés de benzopyrane et leurs produits d'addition d'acide acceptables d'un point de vue pharmacologique selon la revendication 5, dans lesquels le groupe substituant au niveau de la position 7 du cycle benzopyrane est un groupe alcoxy en $C_7$ à $C_{20}$ substitué ou non substitué.

**7.** Dérivés de benzopyrane et leurs produits d'addition d'acide acceptables d'un point de vue pharmacologique selon la revendication 1, dans lesquels n dans la formule (I) vaut 0 et $R^8$ est le groupe de formule (IX).

**8.** Dérivés de benzopyrane et leurs produits d'addition d'acide acceptables d'un point de vue pharmacologique selon la revendication 7, dans lesquels la position de substitution du groupe Z est la position 8 du cycle benzopyrane.

**9.** Dérivés de benzopyrane et leurs produits d'addition d'acide acceptables d'un point de vue pharmacologique selon la revendication 8, dans lesquels il y a un groupe substituant autre que l'atome d'hydrogène au niveau de la position 7 du cycle benzopyrane.

**10.** Composition pharmaceutique comprenant une quantité efficace d'un dérivé de benzopyrane ou d'un de ses produits d'addition d'acide acceptables d'un point de vue pharmacologique selon l'une quelconque des revendications 1 à 9, et un véhicule acceptable d'un point de vue pharmacologique.

**11.** Inhibiteur de l'ACAT sous forme posologique unitaire ayant, à titre d'ingrédient principal, une quantité efficace inhibitrice de l'ACAT d'un dérivé de benzopyrane ou d'un de ses produits d'addition d'acide acceptables d'un point de vue pharmacologique selon l'une quelconque des revendications 1 à 9, et un véhicule acceptable d'un point de vue pharmacologique.

**12.** Médicament pour l'artériosclérose sous forme posologique unitaire ayant, à titre d'ingrédient principal, une quantité

efficace anti-artériosclérose d'un dérivé de benzopyrane ou d'un de ses produits d'addition d'acide acceptables d'un point de vue pharmacologique selon l'une quelconque des revendications 1 à 9, et un véhicule acceptable d'un point de vue pharmacologique.

**13.** Médicament pour l'hyperlipidémie sous forme posologique unitaire ayant, à titre d'ingrédient principal, une quantité efficace anti-hyperlipidémie d'un dérivé de benzopyrane ou d'un de ses produits d'addition d'acide acceptables d'un point de vue pharmacologique selon l'une quelconque des revendications 1 à 9, et un véhicule acceptable d'un point de vue pharmacologique.